# EUROPEAN PATENT APPLICATION

(11) **EP 4 226 942 A1**
(43) Date of publication of application: **16.08.2023**
(21) Application number: 21877032.9
(22) Date of filing: 09.10.2021
(51) Int. Cl.: A61K 39/395, A61K 31/537, C07K 16/28, A61P 35/00

(54) **USE OF ANTI-PD-1 ANTIBODY IN COMBINATION MEDICATION**

(30) Priority: 11.10.2020 WO PCT/CN2020/120262
(71) Applicant: Bio-Thera Solutions, Ltd., Guangzhou, Guangdong 510530 (CN)
(72) Inventor: YUE, Haitao, Guangzhou, Guangdong 510530 (CN); PEI, Shujun, Guangzhou, Guangdong 510530 (CN); SU, Ziqi, Guangzhou, Guangdong 510530 (CN); TANG, Weijia, Guangzhou, Guangdong 510530 (CN); YU, Jin-Chen, Guangzhou, Guangdong 510530 (CN); LI, Shengfeng, Guangzhou, Guangdong 510530 (CN)
(74) Representative: Engelhard, Markus
(86) International application number: PCT/CN2021/122878
(87) International publication number: WO 2022/073515

(57) **Abstract**

The present invention discloses use of an anti-PD-1 antibody in a combination therapy. A treatment method comprises administering to a patient in need an effective amount of an anti-PD-1 antibody and a therapeutic agent.

## Description

### TECHNICAL FIELD

The present invention belongs to the field of bio-pharmaceuticals, and particularly relates to use of an anti-PD-1 antibody in combination therapy.

### BACKGROUND

The incidence of tumors is increasing year by year, and the use of anti-tumor drugs is also gradually increasing. In recent years, significant progress has been made in tumor treatment researches, and especially some antibody drugs have shown good efficacy in the treatment of malignant tumors. However, only a small proportion of patients can achieve long-term survival. In order to further improve the clinical efficacy of drugs in the anti-tumor treatment, the anti-tumor drugs may be used in combination therapy.

Programmed cell death receptor-1 (PD-1) is an immunoinhibitory receptor expressed on activated T cells, B cells, and myeloid cells, and is a member of the CD28 immunoglobulin superfamily. PD-1 is a 55 kDa type I transmembrane glycoprotein comprising an Ig variable domain for binding to a ligand, and a cytoplasmic tail responsible for binding to a signaling molecule. The PD-1 cytoplasmic tail contains two tyrosine-based signaling motifs, immunoreceptor tyrosine-based inhibitory motif (ITIM), and immunoreceptor tyrosine-based switch motif (ITSM).

To date, numerous studies have shown that the interaction between PD-1 and PD-L1 (programmed death ligand-1) results in a decrease in lymphocytes infiltrating the tumor, a decrease in T cell receptor-mediated proliferation, and immune evasion of cancer cells. Blocking the interaction between PD-1 and PD-L1 can increase T cell proliferation and cytokine production, and improve the immunity of tumor-specific CD8+ T cells.

Therefore, the anti-PD-1 antibody plays an important role in anti-tumor combination therapy.

### SUMMARY

The present invention discloses a method or use of an anti-PD-1 antibody for treating a tumor or cancer in combination. In some embodiments, the anti-PD-1 antibody is used in combination with a therapeutic agent for treating a tumor or cancer. In some embodiments, the therapeutic agent is an antibody or an antibody-drug conjugate.

In some embodiments, the anti-PD-1 antibody comprises at least one or more of an HCDR1 set forth in SEQ ID NO: 1, an HCDR2 set forth in SEQ ID NO: 2, an HCDR3 set forth in SEQ ID NO: 3, an LCDR1 set forth in SEQ ID NO: 4, an LCDR2 set forth in SEQ ID NO: 5, and an LCDR3 set forth in SEQ ID NO: 6.

In some embodiments, the anti-PD-1 antibody comprises an HCDR1 set forth in SEQ ID NO: 1, an HCDR2 set forth in SEQ ID NO: 2, an HCDR3 set forth in SEQ ID NO: 3, an LCDR1 set forth in SEQ ID NO: 4, an LCDR2 set forth in SEQ ID NO: 5, and an LCDR3 set forth in SEQ ID NO: 6.

In some embodiments, the anti-PD-1 antibody comprises a heavy chain variable region comprising a sequence set forth in SEQ ID NO: 7, a sequence having at least 80% identity to the sequence set forth in SEQ ID NO: 7, or an amino acid sequence having one or more conservative amino acid substitutions as compared to the sequence set forth in SEQ ID NO: 7; and/or
the anti-PD-1 antibody comprises a light chain variable region comprising a sequence set forth in SEQ ID NO: 8, a sequence having at least 80% identity to the sequence set forth in SEQ ID NO: 8, or an amino acid sequence having one or more conservative amino acid substitutions as compared to the sequence set forth in SEQ ID NO: 8.

In some embodiments, the anti-PD-1 antibody comprises a heavy chain variable region comprising a sequence set forth in SEQ ID NO: 7, and a light chain variable region comprising a sequence set forth in SEQ ID NO: 8.

In some embodiments, the anti-PD-1 antibody comprises a heavy chain variable region comprising a sequence set forth in SEQ ID NO: 9, a sequence having at least 80% identity to the sequence set forth in SEQ ID NO: 9, or an amino acid sequence having one or more conservative amino acid substitutions as compared to the sequence set forth in SEQ ID NO: 9; and/or
the anti-PD-1 antibody comprises a light chain comprising a sequence set forth in SEQ ID NO: 10, a sequence having at least 80% identity to the sequence set forth in SEQ ID NO: 10, or an amino acid sequence having one or more conservative amino acid substitutions as compared to the sequence set forth in SEQ ID NO: 10.

In some embodiments, the anti-PD-1 antibody is an antibody A, wherein the antibody A comprises a heavy chain comprising a sequence set forth in SEQ ID NO: 9, and a light chain comprising a sequence set forth in SEQ ID NO: 10; and the antibody A comprises two heavy chains with identical sequences and two light chains with identical sequences.

In some embodiments, the therapeutic agent is selected from antibodies or antibody-drug conjugates (ADCs) directed against the following targets: EGFR (epidermal growth factor receptor), VEGF (vascular endothelial growth factor), VEGFR2 (vascular endothelial growth factor receptor 2), CTLA4 (cytotoxic T lymphocyte-associated protein 4), PD-L1, HER2 (human epidermal growth factor receptor 2), CD20 (cluster of differentiation 20), Trop2 (human trophoblast cell-surface antigen 2), Lag3 (lymphocyte-activation gene-3), TIGIT (T cell Ig and ITIM domain), CD27 (cluster of differentiation 27), OX40 (tumor necrosis factor receptor superfamily member 4), ICOS (inducible costimulator), BTLA (B and T lymphocyte attenuator), TIM3 (T cell immunoglobulin mucin 3), BCMA (B cell maturation antigen), c-MET (c-mesenchymal to epithelial transition factor), and TAA-1/2/3 (tumor-associated antigen). In some embodiments, the antibody is an inhibitory antibody or an agonistic antibody.

In some embodiments, the therapeutic agent is selected from an anti-EGFR antibody, an anti-VEGF antibody, an anti-VEGFR2 antibody, an anti-CTLA4 antibody, an anti-PD-L1 antibody, an anti-HER2 antibody, an anti-CD20 antibody, an anti-Trop2 antibody, an anti-TIGIT antibody, an anti-OX40 antibody, and an anti-ICOS antibody.

In some embodiments, the therapeutic agent is an anti-CTLA4 antibody. In some embodiments, the anti-CTLA4 antibody is an antibody C, wherein the antibody C comprises a heavy chain comprising a sequence set forth in SEQ ID NO: 15, and a light chain comprising a sequence set forth in SEQ ID NO: 16; and the antibody C comprises two heavy chains with identical sequences and two light chain with identical sequences. In some embodiments, the antibody C is expressed by α-(1,6)-fucosyltransferase gene knock-out CHO cells, such as a CHO-BAT-KF cell line disclosed in PCT/CN2018/100008.

In some embodiments, the Fc region in the antibody C has high mannose glycoforms with a total amount of < 5% and/or sialylated glycoforms with a total amount of < 3%. In some embodiments, the Fc region in the antibody C has high mannose glycoforms with a total amount of about 0.1%, about 0.3%, about 0.9%, about 1.18%, about 1.7%, about 2.6%, about 3.3%, about 4.1%, about 4.9%, about 4.99%, or a range between any two of these values (inclusive) or any value therein. In some embodiments, the Fc region in the antibody C has sialylated glycoforms with a total amount of about 0.1%, about 0.2%, about 0.36%, about 0.8%, about 1.5%, about 2.2%, about 2.7%, about 2.9%, 2.99%, or a range between any two of these values (inclusive) or any value therein.

In some embodiments, the Fc region in the antibody C has high mannose glycoforms with a total amount of < 2% and/or sialylated glycoforms with a total amount of < 1%.

In some embodiments, the Fc region in the antibody C has a fucosylation level of 0%-10%. In some embodiments, the Fc region in the antibody C has a fucosylation level of 0%-5%. In some embodiments, the Fc region in the antibody C has a fucosylation content of about 0, about 0.1%, about 0.3%, about 0.4%, about 0.6%, about 1.3%, about 1.9%, about 2.2%, about 2.8%, about 3.3%, about 3.7%, about 4.1%, about 4.5%, about 5%, or a range between any two of these values (inclusive) or any value therein.

In some embodiments, the therapeutic agent is an anti-HER2 antibody-drug conjugate or an anti-Trop2 antibody-drug conjugate. In some embodiments, the therapeutic agent is an anti-HER2 antibody-drug conjugate (HER2-ADC). In some embodiments, the therapeutic agent is an anti-Trop2 antibody-drug conjugate (Trop2-ADC).

In some embodiments, the Trop2-ADC is a conjugate of BAT0807 or BAT0808 as described in the patent application CN109078181A with a drug.

In some embodiments, the therapeutic agent is an antibody-drug conjugate (ADC) of formula I or a pharmaceutically acceptable salt thereof: 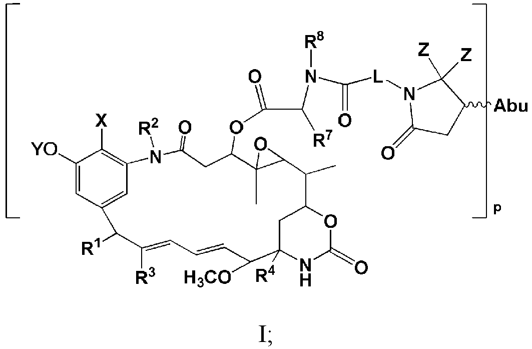
wherein, Abu is an anti-HER2 antibody or an anti-Trop2 antibody, and p is selected from 1-10;
X is -H or halogen;
Y is selected from the group consisting of -H, C1-C6 alkyl, C3-C6 cycloalkyl, and -C(=O)R⁵;
R¹ is selected from the group consisting of -H, -OH, -OC(=O)R⁵, and -OR⁵;
R² is -H or C1-C6 alkyl;
R³ is methyl, -CH₂OH, or -CH₂OC(=O)R⁶;
R⁴ is -OH or -SH;
R⁵ is C1-C6 alkyl or benzyl;
R⁶ is C1-C6 alkyl, phenyl, or benzyl;
R⁷ is -H, C1-C6 alkyl, or an amino acid side chain;
R⁸ is -H or C1-C6 alkyl;
Z is independently -H or C1-C4 hydrocarbyl, or two Z, together with the carbon atom to which they are attached, form carbonyl;
L is selected from optionally substituted C1-C20 hydrocarbylene or C3-C8 cyclohydrocarbylene, wherein one or more -CH₂- groups are independently and optionally substituted with groups selected from the following: C3-C8 cyclohydrocarbylene, -O-, -S-,-NR⁸-, -C(=O)-, -C(=O)NR⁸-, -NR⁸C(=O)-, -SO₂NR⁸-, and -NR⁸SO₂-.

In some embodiments, L is -(CH₂)ₘ-, wherein m is an integer and m is 1-20. In some embodiments, m is 1-10. In some embodiments, m is 5-10. In some embodiments, m is 1, 2, 3, 4, 5, 7, 9, 11, 12, 13, 15, 16, 18, 19, or 20, or a range between any two of these values (inclusive) or any value therein.

In some embodiments, the C1-C20 hydrocarbylene in L is substituted with 1 to 4 -SO₃H, - P(=O)(OH)₂, or R²³, wherein R²³ are each independently C1-C6 alkyl optionally substituted with one or two groups independently selected from -SH, -S-C₁₋₄ alkyl, -CONR₁₁R₁₁, and - NR₁₁R₁₁. In some embodiments, R₁₁ is selected from -H, alkyl, aryl, cycloalkyl, cycloalkenyl, heteroaryl, and a heterocyclic ring, or two R₁₁, together with a nitrogen atom, form a heterocyclic ring, wherein carbon atoms in the heterocyclic ring may be optionally substituted with one or two oxygen atoms.

In some embodiments, the therapeutic agent is an antibody-drug conjugate (ADC) of formula II or a pharmaceutically acceptable salt thereof: 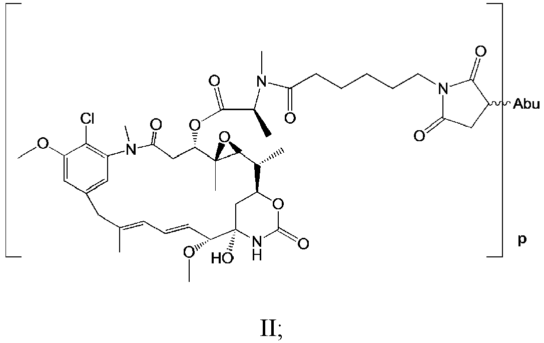
wherein, Abu is an anti-HER2 antibody or an anti-Trop2 antibody, and p is selected from 1-10.
p is the number of small molecule drugs binding to one antibody in the ADC, i.e., the number of antibody-binding drugs, or referred to as the drug to antibody ratio (DAR). In some embodiments, p is about 1, about 2, about 3, about 4, about 5, about 6, about 7, about 8, about 9, or about 10. In some embodiments, p is 2-3. In some embodiments, p is about 2.

In some embodiments, p is the mean number of small molecule drugs bound, i.e., the mean number of antibody-binding drugs, or is referred to as the mean drug to antibody ratio (mean DAR). In some embodiments, p is 1-10, and p may be a non-integer. In some embodiments, p is about 1, about 2, about 3, about 4, about 5, about 6, about 7, about 8, about 9, about 10, or a range between any two of these values (inclusive) or any value therein. In some embodiments, p is selected from 2-8. In some embodiments, p is selected from 3-5. In some embodiments, p is selected from 3.3-3.7. In some embodiments, p is about 3.5. In some embodiments, p is about 2.1.

In some embodiments, the anti-HER2 antibody comprises a heavy chain variable region comprising a sequence set forth in SEQ ID NO: 11, a sequence having at least 80% identity to the sequence set forth in SEQ ID NO: 11, or an amino acid sequence having one or more conservative amino acid substitutions as compared to the sequence set forth in SEQ ID NO: 11; and/or

the anti-HER2 antibody comprises a light chain comprising a sequence set forth in SEQ ID NO: 12, a sequence having at least 80% identity to the sequence set forth in SEQ ID NO: 12, or an amino acid sequence having one or more conservative amino acid substitutions as compared to the sequence set forth in SEQ ID NO: 12.

In some embodiments, the anti-HER2 antibody is an antibody B, wherein the antibody B comprises a heavy chain comprising a sequence set forth in SEQ ID NO: 11, and a light chain comprising a sequence set forth in SEQ ID NO: 12.

In some embodiments, the anti-Trop2 antibody is BAT0807 or BAT0808 as described in the patent application CN109078181A. In some embodiments, p is about 2. In some embodiments, p is about 2.1.

In some embodiments, the HER2-ADC is HER2-ADC_{B}, wherein the HER2-ADC_{B} is a compound of formula II, and the antibody in the HER2-ADC_{B} is an antibody B, which comprises two heavy chains with identical sequences and two light chains with identical sequences; and p is 3.3-3.7, or about 3.5.

The antibody protein can be expressed in CHO cells by genetic engineering and obtained by purification. The purification can be performed by conventional methods, for example, by performing centrifugation on a cell suspension and collecting a supernatant, and performing centrifugation again to further remove impurities. The antibody protein can be purified by a protein A affinity column, an ion exchange column, and other methods.

In some embodiments, the method or use comprises: administering to a patient in need an effective amount of an anti-PD-1 antibody and a therapeutic agent. In some embodiments, the anti-PD-1 antibody is an antibody A. In some embodiments, the therapeutic agent is HER2-ADC_{B}. In some embodiments, the anti-PD-1 antibody is administered at an effective dose of about 50 mg to 600 mg per dose. In some embodiments, the HER2-ADC is administered at an effective dose of about 70 mg to 400 mg per dose. In some embodiments, the therapeutic agent is an anti-CTLA4 antibody. In some embodiments, the therapeutic agent is an antibody C. In some embodiments, the anti-CTLA4 is administered at an effective dose of about 6 mg to 600 mg per dose.

In some embodiments, the anti-PD-1 antibody and the therapeutic agent are each separate dosage units administered in combination. In some embodiments, the anti-PD-1 antibody can be administered before, after, or simultaneously with the therapeutic agent.

In some embodiments, the anti-PD-1 antibody and the therapeutic agent simultaneously form combined dosage units administered in combination.

In some embodiments, the patient has a tumor or cancer. In some embodiments, the tumor or cancer include, but are not limited to: head and neck tumor, Hodgkin's lymphoma, non-Hodgkin's lymphoma, pre-lymphoblastic lymphoma, small non-cleaved cell lymphoma, Burkitt's lymphoma, non-Burkitt's lymphoma, diffuse large B cell lymphoma, anaplastic large cell lymphoma, kidney tumor, nephroblastoma, Wilms tumor, clear cell renal cell carcinoma, rhabdoid tumor of the kidney, clear cell sarcoma of the kidney, primitive neuroectodermal tumor of the kidney, neuroblastoma, ganglioneuroblastoma, ganglioneuroma, extracranial germ cell tumor, mature teratoma, immature teratoma, endodermal sinus tumor, yolk sac tumor, seminoma, dysgerminoma, chorionic epithelioma, embryonal carcinoma, osteosarcoma, chondrosarcoma, rhabdomyosarcoma, soft tissue sarcoma, fibrosarcoma, malignant fibrous histiocytoma, liposarcoma, leiomyosarcoma, hemangiosarcoma, lymphangiosarcoma, malignant neurinoma, alveolar soft part sarcoma, epithelioid sarcoma, clear cell sarcoma, malignant melanoma, synovial sarcoma, desmoplastic small round cell tumor, Ewing's sarcoma, primitive neuroectodermal tumor, liver tumor, hepatoblastoma, retinoblastoma, posterior fossa medulloblastoma, thymoma, pulmonary blastoma, pancreatoblastoma, islet cell tumor, ileocecal carcinoid, mesothelioma, melanoma, interstitial cell tumor, myeloma, brain astrocytoma, nasopharyngeal carcinoma, thyroid papillary carcinoma, intestinal cancer, mastocarcinoma, gastric cancer, liver cancer, prostate cancer, breast cancer, lung cancer, cervical cancer, ovarian cancer, renal cancer, lymphoma, leukemia, skin cancer, esophageal squamous carcinoma, and the like.

In some embodiments, the present invention discloses a method for treating a tumor or cancer in a patient in need, which comprises administering an effective amount of an anti-PD-1 antibody and a therapeutic agent, wherein the anti-PD-1 is administered at an effective amount of about 50 mg to 600 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the anti-PD-1 antibody is an antibody A.

In some embodiments, the therapeutic agent is a monoclonal antibody (anti-HER2 antibody) that specifically binds to the extracellular dimerization domain (subdomain II) of epidermal growth factor receptor 2 (HER2), e.g., pertuzumab, having a molecular weight of about 148 kDa. Pertuzumab can be expressed in cells (e.g., CHO) by genetic engineering and obtained by purification. The purification can be performed by conventional methods, for example, by performing centrifugation on a cell suspension and collecting a supernatant, and performing centrifugation again to further remove impurities. The antibody can be purified by a protein A affinity column, an ion exchange column, and other methods.

In some embodiments, the therapeutic agent is pertuzumab, which includes perjeta^{®} or a biosimilar thereof, an ADCC-enhanced monoclonal antibody, or a defucosylated monoclonal antibody. In some embodiments, the pertuzumab is administered at an effective amount of about 40 mg to 900 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the pertuzumab is administered at an effective amount of about 840 mg initially, followed by 420 mg once every 3 weeks.

In some embodiments, the therapeutic agent is a recombinant humanized immunoglobulin G1 (IgG1) monoclonal antibody that can bind to VEGF-A and inhibit its binding to VEGF receptor-2 (VEGFR-2) (anti-VEGF antibody), e.g., bevacizumab. Bevacizumab can be expressed in cells (e.g., CHO) by genetic engineering and obtained by purification. The purification can be performed by conventional methods, for example, by performing centrifugation on a cell suspension and collecting a supernatant, and performing centrifugation again to further remove impurities. The antibody can be purified by a protein A affinity column, an ion exchange column, and other methods.

In some embodiments, the therapeutic agent is bevacizumab, which includes avastin^{®} or a biosimilar thereof (such as Ankeda^{®}, Byvasda^{®}, Mvasi^{®}, Zirabev^{®} or BAT1706), an ADCC-enhanced monoclonal antibody, or a defucosylated monoclonal antibody.

In some embodiments, the bevacizumab is administered at an effective amount of about 50 mg to 400 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the bevacizumab is administered at an effective amount of about 5 mg/kg to 15 mg/kg once every 2 weeks or every 3 weeks. In some embodiments, the bevacizumab is administered at an effective amount of about 5 mg/kg, 7.5 mg/kg, 10 mg/kg, or 15 mg/kg once every 2 weeks or every 3 weeks. In some embodiments, the bevacizumab is administered at an effective amount of about 5 mg/kg once every 2 weeks, 10 mg/kg once every 2 weeks, 7.5 mg/kg once every 3 weeks, or 15 mg/kg once every 3 weeks.

In some embodiments, the therapeutic agent is a CD20-targeted antibody (anti-CD20 antibody), such as ofatumumab (a fully human IgG1 kappa monoclonal antibody) or obinutuzumab. Ofatumumab and obinutuzumab can be expressed in cells (e.g., CHO) by genetic engineering and obtained by purification. The purification can be performed by conventional methods, for example, by performing centrifugation on a cell suspension and collecting a supernatant, and performing centrifugation again to further remove impurities. The antibody can be purified by a protein A affinity column, an ion exchange column, and other methods.

In some embodiments, the therapeutic agent is ofatumumab, which includes Arzerra^{®} or Kesimpta^{®} or a biosimilar thereof, an ADCC-enhanced monoclonal antibody, or a defucosylated monoclonal antibody, such as BAT4406F as disclosed in CN109096399A. In some embodiments, the ofatumumab is administered at an effective amount of about 10 mg to 2000 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the ofatumumab is administered at an effective amount of about 20 mg once every week or month. In some embodiments, the ofatumumab is administered at an effective amount of about 300 mg initially, 1000 mg after 1 week, followed by 1000 mg once every 4 weeks or every 8 weeks. In some embodiments, the ofatumumab is administered at an effective amount of about 300 mg initially, 2000 mg after 1 week, followed by 2000 mg once every 1 week or every 4 weeks.

In some embodiments, the therapeutic agent is obinutuzumab, which includes Gazyva^{®} or a biosimilar thereof, an ADCC-enhanced monoclonal antibody, or a defucosylated monoclonal antibody, such as BAT4306F as described in CN109096399A. In some embodiments, the obinutuzumab is administered at an effective amount of about 10 mg to 2000 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the obinutuzumab is administered at an effective amount of 100 mg on day 1, 900 mg on day 2, 1000 mg on days 8 and 15, followed by 1000 mg per course of treatment. In some embodiments, the obinutuzumab is administered at an effective amount of 1000 mg on each of day 1, day 8 and day 15, followed by 1000 mg per course of treatment. Each course of treatment may be 1 month or 2 months.

In some embodiments, the therapeutic agent is a T lymphocyte-associated antigen 4 (CTLA4)-targeted antibody (anti-CTLA4 antibody), such as ipilimumab, which is an IgG1 kappa immunoglobulin molecule, having a molecular weight of about 148 kDa. Ipilimumab can be expressed in cells (e.g., CHO) by genetic engineering and obtained by purification. The purification can be performed by conventional methods, for example, by performing centrifugation on a cell suspension and collecting a supernatant, and performing centrifugation again to further remove impurities. The antibody can be purified by a protein A affinity column, an ion exchange column, and other methods.

In some embodiments, the therapeutic agent is ipilimumab, which includes Yervoy^{™} or a biosimilar thereof, an ADCC-enhanced monoclonal antibody, or defucosylated ipilimumab, as described in WO2014089113. In some embodiments, the ipilimumab is administered at an effective amount of about 30 mg to 300 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the ipilimumab is administered at an effective amount of 1 mg/kg, 3 mg/kg, or 10 mg/kg once every 3 weeks, 6 weeks, or 12 weeks. In some embodiments, the ipilimumab is administered at an effective amount of 1 mg/kg once every 3 or 6 weeks. In some embodiments, the ipilimumab is administered at an effective amount of 3 mg/kg once every 3 or 6 weeks. In some embodiments, the ipilimumab is administered at an effective amount of 10 mg/kg once every 3 weeks or every 12 weeks.

In some embodiments, the therapeutic agent is Trop2-ADC as described herein. In some embodiments, the Trop2-ADC is administered at an effective amount of about 60 mg to 400 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the Trop2-ADC is administered at an effective amount of 0.2 mg/kg, 0.5 mg/kg, 1 mg/kg, 2 mg/kg, 4 mg/kg, 6 mg/kg, 8 mg/kg or 10 mg/kg once every week to every 3 weeks. In some embodiments, the Trop2-ADC is administered at an effective amount of 0.2 mg/kg, 0.5 mg/kg, 1 mg/kg, 2 mg/kg, 4 mg/kg, 6 mg/kg, 8 mg/kg or 10 mg/kg once every week or every 3 weeks.

In some embodiments, the therapeutic agent is HER2-ADC. In some embodiments, the HER2-ADC is administered at an effective amount of about 70 mg to 400 mg per treatment cycle. In some embodiments, one treatment cycle is 1 week, 2 weeks, 3 weeks, 4 weeks, 1 month, 5 weeks, 6 weeks, 7 weeks, or a range between any two of these values (inclusive) or any value therein. In some embodiments, the HER2-ADC is HER2-ADC_{B}.

In some embodiments, the anti-PD-1 antibody and the therapeutic agent (or a combination of the PD-1 antibody and the therapeutic agent) can be separately formulated into a pharmaceutical composition and administered to the patient in a variety of forms suitable for the selected route of administration, for example, parenteral, intravenous (iv), intramuscular, topical, or subcutaneous route. In some embodiments, the anti-PD-1 antibody and the therapeutic agent (or a combination of the PD-1 antibody and the therapeutic agent) can be separately infused intravenously. The amount of the anti-PD-1 antibody and the therapeutic agent (or formulation) will depend on the properties of the drug, the extent to which the internalization, transport and release of the drug is triggered at the cell surface, the disease being treated, and the conditions of the patient (e.g., age, sex, body weight, and the like).

In some embodiments, the anti-PD-1 antibody at a dose of about 1 mg/kg to 10 mg/kg or a formulation comprising the anti-PD-1 antibody at this dose is administered each time. In some embodiments, the anti-PD-1 antibody at a dose of about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, or a range between any two of these values (inclusive) or any value therein, or a formulation comprising the anti-PD-1 antibody at this dose is administered each time.

In some embodiments, a therapeutically effective amount of pertuzumab and an anti-PD-1 antibody are administered separately or simultaneously to a subject patient. The pertuzumab and the anti-PD-1 antibody may have identical or different administration cycles.

In some embodiments, pertuzumab at a dose of about 1 mg/kg to 12 mg/kg or a formulation comprising pertuzumab at this dose is administered each time. In some embodiments, pertuzumab at a dose of at about 1 mg/kg, about 1.2 mg/kg, about 2 mg/kg, about 2.4 mg/kg, about 3 mg/kg, about 3.6 mg/kg, about 4 mg/kg, about 4.8 mg/kg, about 5 mg/kg, about 5.5 mg/kg, about 6 mg/kg, about 6.9 mg/kg, about 7 mg/kg, about 8.4 mg/kg, about 9 mg/kg, about 11 mg/kg, about 12 mg/kg, or a range between any two of these values (inclusive) or any value therein, or a formulation comprising pertuzumab at this dose is administered each time.

In some embodiments, a therapeutically effective amount of bevacizumab and an anti-PD-1 antibody are administered separately or simultaneously to a subject patient. The bevacizumab and the anti-PD-1 antibody may have identical or different administration cycles.

In some embodiments, bevacizumab at a dose of about 1 mg/kg to 9 mg/kg or a formulation comprising bevacizumab at this dose is administered each time. In some embodiments, bevacizumab at a dose of about 1 mg/kg, about 1.2 mg/kg, about 2 mg/kg, about 2.4 mg/kg, about 3mg/kg, about 3.6 mg/kg, about 4 mg/kg, about 4.8 mg/kg, about 5 mg/kg, about 5.5 mg/kg, about 6 mg/kg, about 6.9 mg/kg, about 7 mg/kg, about 8.4 mg/kg, about 9 mg/kg, or a range between any two of these values (inclusive) or any value therein, or a formulation comprising bevacizumab at this dose is administered each time.

In some embodiments, a therapeutically effective amount of ofatumumab and an anti-PD-1 antibody are administered separately or simultaneously to a subject patient. The ofatumumab and the anti-PD-1 antibody may have identical or different administration cycles.

In some embodiments, ofatumumab at a dose of about 0.5 mg/kg to 18 mg/kg or a formulation comprising ofatumumab at this dose is administered each time. In some embodiments, ofatumumab at a dose of about 0.5 mg/kg, about 1 mg/kg, about 1.2 mg/kg, about 2 mg/kg, about 2.4 mg/kg, about 3 mg/kg, about 3.6 mg/kg, about 4 mg/kg, about 4.8 mg/kg, about 5 mg/kg, about 5.5 mg/kg, about 6 mg/kg, about 6.9 mg/kg, about 7 mg/kg, about 8.4 mg/kg, about 9 mg/kg, about 11 mg/kg, about 13 mg/kg, about 14 mg/kg, about 15 mg/kg, about 17 mg/kg, about 18 mg/kg, or a range between any two of these values (inclusive), or a formulation comprising ofatumumab at this dose is administered each time.

In some embodiments, a therapeutically effective amount of obinutuzumab and an anti-PD-1 antibody are administered separately or simultaneously to a subject patient. The obinutuzumab and the anti-PD-1 antibody may have identical or different administration cycles.

In some embodiments, obinutuzumab at a dose of about 0.5 mg/kg to 15 mg/kg or a formulation comprising obinutuzumab at this dose is administered each time. In some embodiments, ofatumumab at a dose of about 0.5 mg/kg, about 1 mg/kg, about 1.2 mg/kg, about 2 mg/kg, about 2.4 mg/kg, about 3 mg/kg, about 3.6 mg/kg, about 4 mg/kg, about 4.8 mg/kg, about 5 mg/kg, about 5.5 mg/kg, about 6 mg/kg, about 6.9 mg/kg, about 7 mg/kg, about 8.4 mg/kg, about 9 mg/kg, about 11 mg/kg, about 13 mg/kg, about 14 mg/kg, about 15 mg/kg, or a range between any two of these values (inclusive) or any value therein, or a formulation comprising ofatumumab at this dose is administered each time.

In some embodiments, a therapeutically effective amount of an anti-CTLA4 antibody (e.g., ipilimumab or defucosylated ipilimumab) and an anti-PD-1 antibody are administered separately or simultaneously to a subject patient. The anti-CTLA4 antibody (e.g., ipilimumab or defucosylated ipilimumab) and the anti-PD-1 antibody may have identical or different administration cycles.

In some embodiments, the anti-CTLA4 antibody (e.g., ipilimumab or defucosylated ipilimumab) at a dose of about 0.1 mg/kg to 10 mg/kg or about 0.5 mg/kg to 10 mg/kg, or a formulation comprising the anti-CTLA4 antibody (e.g., ipilimumab or defucosylated ipilimumab) at this dose is administered each time. In some embodiments, ipilimumab at dose of about 0.5 mg/kg, about 1 mg/kg, about 1.2 mg/kg, about 2 mg/kg, about 2.4 mg/kg, about 3 mg/kg, about 3.6 mg/kg, about 4 mg/kg, about 4.8 mg/kg, about 5 mg/kg, about 5.5 mg/kg, about 6 mg/kg, about 6.9 mg/kg, about 7 mg/kg, about 8.4 mg/kg, about 9 mg/kg, about 10 mg/kg, or a range between any two of these values (inclusive) or any value therein, or a formulation comprising ipilimumab at this dose is administered each time.

In some embodiments, a therapeutically effective amount of a Trop2-ADC and an anti-PD-1 antibody are administered to a subject patient separately or simultaneously. The Trop2-ADC and the anti-PD-1 antibody may have identical or different administration cycles.

In some embodiments, the Trop2-ADC at a dose of about 0.5 mg/kg to 7 mg/kg or a formulation comprising the Trop2-ADC at this dose is administered each time. In some embodiments, the Trop2-ADC at a dose of about 1 mg/kg, about 1.2 mg/kg, about 2 mg/kg, about 2.4 mg/kg, about 3 mg/kg, about 3.6 mg/kg, about 4 mg/kg, about 4.8 mg/kg, about 5 mg/kg, about 5.5 mg/kg, about 7 mg/kg, or a range between any two of these values (inclusive) or any value therein, or a formulation comprising the Trop2-ADC at this dose is administered each time.

In some embodiments, a therapeutically effective amount of HER2-ADC and an anti-PD-1 antibody are administered to a subject patient separately or simultaneously. The HER2-ADC and the anti-PD-1 antibody may have identical or different administration cycles.

In some embodiments, the HER2-ADC at a dose of about 1 mg/kg to 6 mg/kg or a formulation comprising the HER2-ADC at this dose is administered each time. In some embodiments, the HER2-ADC at a dose of about 1 mg/kg, about 1.2 mg/kg, about 2 mg/kg, about 2.4 mg/kg, about 3 mg/kg, about 3.6 mg/kg, about 4 mg/kg, about 4.8 mg/kg, about 5 mg/kg, about 5.5 mg/kg, about 6 mg/kg, or a range between any two of these values (inclusive) or any value therein, or a formulation comprising the HER2-ADC at this dose is administered each time. In some embodiments, the present invention discloses a method for treating a tumor or cancer, which comprises administering to a patient in need an effective amount of an anti-PD-1 antibody (or formulation) and a therapeutic agent (or formulation), wherein the anti-PD-1 antibody at an effective amount of about 50 mg to 600 mg (or a formulation comprising the anti-PD-1 antibody at this dose) is administered each time. In some embodiments, the therapeutic agent is HER2-ADC. In some embodiments, the HER2-ADC at an effective amount of about 70 mg to 400 mg (or a formulation comprising the HER2-ADC at this dose) is administered each time. In some embodiments, the therapeutic agent is an anti-CTLA4 antibody. In some embodiments, the anti-CTLA4 antibody is an antibody C. In some embodiments, the anti-CTLA4 antibody at an effective amount of about 6 mg to 600 mg (or a formulation comprising the anti-CTLA4 antibody at this dose) is administered each time. The dosage schedule and mode of administration will depend on the benefit-risk assessment and general clinical practice guidelines for the anti-PD-1 antibody (or formulation), the HER2-ADC (or formulation), or the anti-CTLA4 antibody (or formulation) in certain patient populations.

In some embodiments, the anti-PD-1 antibody at an effective amount of about 50 mg to 600 mg (or a formulation comprising the anti-PD-1 antibody at this dose) is administered to the patient per treatment cycle, and the HER2-ADC at an effective amount of about 70 mg to 400 mg (or a formulation comprising the HER2-ADC at this dose) is administered to the patient per treatment cycle.

In some embodiments, the anti-PD-1 antibody at an effective amount of about 50 mg to 600 mg (or a formulation comprising the anti-PD-1 antibody at this dose) is administered to the patient per treatment cycle, and the anti-CTLA4 antibody at an effective amount of about 6 mg to 600 mg (or a formulation comprising the anti-CTLA4 antibody at this dose) is administered to the patient per treatment cycle.

In some embodiments, the anti-PD-1 antibody at an effective amount of about 50 mg, about 60 mg, about 80 mg, about 120 mg, about 200 mg, about 250 mg, about 290 mg, about 300 mg, about 330 mg, about 380 mg, about 400 mg, about 434 mg, about 480 mg, about 500 mg, about 567 mg, about 580 mg, about 600 mg, or a range between any two of these values (inclusive) or any value therein, or a formulation comprising the anti-PD-1 antibody at this dose is administered to the patient per treatment cycle. In some embodiments, one treatment cycle is administering once every 1 week to 7 weeks. In some embodiments, the anti-PD-1 antibody at an effective amount of 100 mg to 200 mg or a formulation comprising the anti-PD-1 antibody at this dose is administered per treatment cycle; wherein one treatment cycle is about 1 week, about 2 weeks, about 3 weeks, about 4 weeks, about 5 weeks, about 6 weeks, about 7 weeks, or a range between any two of these values (inclusive) or any value therein. In some embodiments, one treatment cycle is about 1 week, about 2 weeks, about 3 weeks, or about 4 weeks. In some embodiments, the anti-PD-1 antibody at an effective amount of about 200 mg to about 300 mg or a formulation comprising the anti-PD-1 antibody at this dose is administered to the patient per treatment cycle; wherein one treatment cycle is about 1 week, about 2 weeks, about 3 weeks, or about 4 weeks. In some embodiments, the anti-PD-1 antibody at an effective amount of about 100 mg, about 110 mg, about 120 mg, about 130 mg, about 140 mg, about 150 mg, about 160 mg, about 170 mg, about 180 mg, about 190 mg, about 200 mg, or a range between any two of these values (inclusive) or any value therein, or a formulation comprising the anti-PD-1 antibody at this dose is administered to the patient per treatment cycle; wherein one treatment cycle is about 1 week, about 2 weeks, about 3 weeks, or about 4 weeks.

In some embodiments, the anti-PD-1 antibody at an effective amount of about 100 mg or a formulation comprising the anti-PD-1 antibody at this dose is administered to the patient per treatment cycle; wherein one treatment cycle is about 1 week, about 2 weeks, about 3 weeks, or about 4 weeks. In some embodiments, the anti-PD-1 antibody at an effective amount of about 90 mg to 110 mg, or a formulation comprising the anti-PD-1 antibody at this dose is administered to the patient per treatment cycle; for example, about 100 mg of the anti-PD-1 antibody is administered once.

In some embodiments, the anti-PD-1 antibody at an effective amount of about 120 mg or a formulation comprising the anti-PD-1 antibody at this dose is administered to the patient per treatment cycle; wherein one treatment cycle is about 1 week, about 2 weeks, about 3 weeks, or about 4 weeks. In some embodiments, the anti-PD-1 antibody at an effective amount of about 100 mg to 140 mg, or a formulation comprising the anti-PD-1 antibody at this dose is administered to the patient per treatment cycle; for example, about 120 mg of the anti-PD-1 antibody is administered once.

In some embodiments, the anti-PD-1 antibody at an effective amount of about 160 mg or a formulation comprising the anti-PD-1 antibody at this dose is administered to the patient per treatment cycle; wherein one treatment cycle is about 1 week, about 2 weeks, about 3 weeks, or about 4 weeks. In some embodiments, the anti-PD-1 antibody at an effective amount of about 150 mg to 190 mg, or a formulation comprising the anti-PD-1 antibody at this dose is administered to the patient per treatment cycle; for example, about 160 mg of the anti-PD-1 antibody is administered once.

In some embodiments, the anti-PD-1 antibody at an effective amount of about 200 mg or a formulation comprising the anti-PD-1 antibody at this dose is administered to the patient per treatment cycle; wherein one treatment cycle is about 1 week, about 2 weeks, about 3 weeks, or about 4 weeks. In some embodiments, the anti-PD-1 antibody at an effective amount of about 190 mg to 230 mg, or a formulation comprising the anti-PD-1 antibody at this dose is administered to the patient per treatment cycle; for example, about 200 mg of the anti-PD-1 antibody is administered once.

In some embodiments, the anti-PD-1 antibody at an effective amount of about 100 mg to 600 mg is administered once every 3 weeks. In some embodiments, the anti-PD-1 antibody at an effective amount of about 100 mg, about 200 mg, about 300 mg, about 400 mg, about 500 mg, or about 600 mg is administered once every 3 weeks. In some embodiments, the anti-PD-1 antibody at an effective amount of about 100 mg, about 300 mg, or about 600 mg is administered once every 3 weeks.

In some embodiments, the HER2-ADC at an effective amount of about 70 mg, about 90 mg, about 120 mg, about 160 mg, about 180 mg, about 200 mg, about 230 mg, about 250 mg, about 280 mg, about 300 mg, about 310 mg, about 334 mg, about 350 mg, about 370 mg, about 380 mg, about 390 mg, about 400 mg, or a range between any two of these values (inclusive) or any value therein, or a formulation comprising the HER2-ADC at this dose is administered to the patient per treatment cycle. In some embodiments, one treatment cycle is administering once every 1 week to 4 weeks. In some embodiments, the HER2-ADC at an effective amount of 100 mg to 200 mg or a formulation comprising the HER2-ADC at this dose is administered per treatment cycle; wherein one treatment cycle is about 1 week, about 2 weeks, about 3 weeks, about 4 weeks, or a range between any two of these values (inclusive) or any value therein. In some embodiments, the HER2-ADC at an effective amount of about 200 mg to about 300 mg or a formulation comprising the HER2-ADC at this dose is administered to the patient per treatment cycle; wherein one treatment cycle is about 1 week, about 2 weeks, about 3 weeks, or about 4 weeks. In some embodiments, the HER2-ADC at an effective amount of about 100 mg, about 110 mg, about 120 mg, about 130 mg, about 140 mg, about 150 mg, about 160 mg, about 170 mg, about 180 mg, about 190 mg, about 200 mg, or a range between any two of these values (inclusive) or any value therein, or a formulation comprising the HER2-ADC at this dose is administered to the patient per treatment cycle; wherein one treatment cycle is about 1 week, about 2 weeks, about 3 weeks, or about 4 weeks.

In some embodiments, the HER2-ADC at an effective amount of about 100 mg or a formulation comprising the HER2-ADC at this dose is administered to the patient per treatment cycle; wherein one treatment cycle is about 1 week, about 2 weeks, about 3 weeks, or about 4 weeks. In some embodiments, the HER2-ADC at an effective amount of about 90 mg to 110 mg or a formulation comprising the HER2-ADC at this dose is administered to the patient per treatment cycle; for example, about 100 mg of the HER2-ADC is administered once.

In some embodiments, the HER2-ADC at an effective amount of about 130 mg or a formulation comprising the HER2-ADC at this dose is administered to the patient per treatment cycle; wherein one treatment cycle is about 1 week, about 2 weeks, about 3 weeks, or about 4 weeks. In some embodiments, the HER2-ADC at an effective amount of between about 100 mg and 140 mg or a formulation comprising the HER2-ADC at this dose is administered to the patient per treatment cycle; for example, about 130 mg of the HER2-ADC is administered once.

In some embodiments, the HER2-ADC at an effective amount of about 170 mg or a formulation comprising the HER2-ADC at this dose is administered to the patient per treatment cycle; wherein one treatment cycle is about 1 week, about 2 weeks, about 3 weeks, or about 4 weeks. In some embodiments, the HER2-ADC at an effective amount of about 150 mg to 190 mg or a formulation comprising the HER2-ADC at this dose is administered to the patient per treatment cycle; for example, about 170 mg of the HER2-ADC is administered once.

In some embodiments, the HER2-ADC at an effective amount of about 200 mg or a formulation comprising the HER2-ADC at this dose is administered to the patient per treatment cycle; wherein one treatment cycle is about 1 week, about 2 weeks, about 3 weeks, or about 4 weeks. In some embodiments, the HER2-ADC at an effective amount of about 190 mg and 230 mg or a formulation comprising the HER2-ADC at this dose is administered to the patient per treatment cycle; for example, about 200 mg of the HER2-ADC is administered once.

In some embodiments, the anti-CTLA4 antibody at an effective amount of about 6 mg, about 8 mg, about 10 mg, about 15 mg, about 20 mg, about 25 mg, about 30 mg, about 35 mg, about 40 mg, about 50 mg, about 60 mg, about 80 mg, about 120 mg, about 200 mg, about 250 mg, about 290 mg, about 300 mg, about 330 mg, about 380 mg, about 400 mg, about 434 mg, about 480 mg, about 500 mg, about 567 mg, about 580 mg, about 600 mg, or a range between any two of these values (inclusive) or any value therein, or a formulation comprising the anti-CTLA4 antibody at this dose is administered to the patient per treatment cycle. In some embodiments, one treatment cycle is administering once every 1 week to 7 weeks. In some embodiments, the anti-CTLA4 antibody at an effective amount of 6 mg to 80 mg or a formulation comprising the anti-CTLA4 antibody at this dose is administered per treatment cycle; wherein one treatment cycle is about 1 week, about 2 weeks, about 3 weeks, about 4 weeks, about 5 weeks, about 6 weeks, about 7 weeks, or a range between any two of these values (inclusive) or any value therein. In some embodiments, the anti-CTLA4 antibody at an effective amount of 100 mg to 200 mg or a formulation comprising the anti-CTLA4 antibody at this dose is administered per treatment cycle; wherein one treatment cycle is about 1 week, about 2 weeks, about 3 weeks, about 4 weeks, about 5 weeks, about 6 weeks, about 7 weeks, or a range between any two of these values (inclusive) or any value therein. In some embodiments, one treatment cycle is about 1 week, about 2 weeks, about 3 weeks, or about 4 weeks. In some embodiments, the anti-CTLA4 antibody at an effective amount of about 200 mg to about 300 mg or a formulation comprising the anti-CTLA4 antibody at this dose is administered to the patient per treatment cycle; wherein one treatment cycle is about 1 week, about 2 weeks, about 3 weeks, or about 4 weeks. In some embodiments, the anti-CTLA4 antibody at an effective amount of about 6 mg, about 8 mg, about 10 mg, about 15 mg, about 20 mg, about 25 mg, about 30 mg, about 35 mg, about 40 mg, about 50 mg, about 60 mg, 100 mg, about 110 mg, about 120 mg, about 130 mg, about 140 mg, about 150 mg, about 160 mg, about 170 mg, or a range between any two of these values (inclusive) or any value therein, or a formulation comprising the anti-CTLA4 antibody at this dose is administered to the patient per treatment cycle; wherein one treatment cycle is about 1 week, about 2 weeks, about 3 weeks, or about 4 weeks.

In some embodiments, the anti-CTLA4 antibody at an effective amount of about 6 mg or a formulation comprising the anti-CTLA4 antibody at this dose is administered to the patient per treatment cycle; wherein one treatment cycle is about 1 week, about 2 weeks, about 3 weeks, or about 4 weeks. In some embodiments, about 6 mg of the anti-CTLA4 antibody is administered to the patient once per treatment cycle.

In some embodiments, the anti-CTLA4 antibody at an effective amount of about 15 mg or a formulation comprising the anti-CTLA4 antibody at this dose is administered to the patient per treatment cycle; wherein one treatment cycle is about 1 week, about 2 weeks, about 3 weeks, or about 4 weeks. In some embodiments, about 15 mg of the anti-CTLA4 antibody is administered to the patient once per treatment cycle.

In some embodiments, the anti-CTLA4 antibody at an effective amount of about 23 mg or a formulation comprising the anti-CTLA4 antibody at this dose is administered to the patient per treatment cycle; wherein one treatment cycle is about 1 week, about 2 weeks, about 3 weeks, or about 4 weeks. In some embodiments, about 23 mg of the anti-CTLA4 antibody is administered to the patient once per treatment cycle.

In some embodiments, the anti-CTLA4 antibody at an effective amount of about 60 mg or a formulation comprising the anti-CTLA4 antibody at this dose is administered to the patient per treatment cycle; wherein one treatment cycle is about 1 week, about 2 weeks, about 3 weeks, or about 4 weeks. In some embodiments, about 60 mg of the anti-CTLA4 antibody is administered to the patient once per treatment cycle.

In some embodiments, the anti-CTLA4 antibody at an effective amount of about 6 mg to 600 mg is administered once every 3 weeks. In some embodiments, the anti-CTLA4 antibody at an effective amount of about 6 mg, about 20 mg, about 30 mg, about 100 mg, about 200 mg, about 230 mg, about 300 mg, about 400 mg, about 500 mg, or about 600 mg is administered once every 3 weeks. In some embodiments, the anti-CTLA4 antibody at an effective amount of about 6 mg, about 30 mg, or about 60 mg is administered once every 3 weeks.

In some embodiments, the anti-PD-1 antibody and the therapeutic agent (or a combination of the anti-PD-1 antibody and the therapeutic agent) are separately administered to the patient once per treatment cycle. In some embodiments, the anti-PD-1 antibody and the therapeutic agent (or a combination of the anti-PD-1 antibody and the therapeutic agent) are separately administered to the patient a plurality of times, e.g., 2 times, 3 times, 4 times, or 5 times, per treatment cycle.

In some embodiments, the patient is subjected to administration only once or 4 times per treatment cycle. In some embodiments, the patient is treated for one treatment cycle. In some embodiments, the patient is treated for a plurality of (e.g., 2, 3, or 4) treatment cycles. In some embodiments, the patient is treated until conditions are alleviated and no treatment is required. In some embodiments, the patient is treated with a plurality of treatment cycles of the anti-PD-1 antibody and 4 treatment cycles of the anti-CTLA4 antibody.

In some embodiments, provided is a method for treating a tumor or cancer, which comprises: administering to a patient in need about 50 mg to 200 mg, about 200 mg to 300 mg, about 300 mg to 400 mg, or about 400 mg to 600 mg, such as about 100 mg, about 120 mg, about 200 mg, or about 400 mg of an anti-PD-1 antibody, or a formulation comprising the anti-PD-1 antibody at this dose; and about 70 mg to 100 mg, about 100 mg to 200 mg, about 200 mg to 300 mg, or about 300 mg to 400 mg, such as about 100 mg, about 120 mg, about 150 mg, or about 200 mg of HER2-ADC, or a formulation comprising the HER2-ADC at this dose. In some embodiments, the patient is treated with a single dose of anti-PD-1 antibody and a single dose of HER2-ADC. In some embodiments, the patient is treated with a single dose of a combination of the anti-PD-1 antibody and the HER2-ADC.

In some embodiments, 200 mg of the anti-PD-1 antibody and 2.4 mg/kg of the HER2-ADC are administered once every 3 weeks. In some embodiments, 200 mg of the anti-PD-1 antibody is administered once every 3 weeks, and 3.6 mg/kg of the HER2-ADC is administered once every 3 weeks.

In some embodiments, provided is a method for treating a tumor or cancer, which comprises: administering to a patient in need about 50 mg to 200 mg, about 200 mg to 300 mg, about 300 mg to 400 mg, or about 400 mg to 600 mg, such as about 100 mg, about 120 mg, about 200 mg, or about 400 mg of an anti-PD-1 antibody, or a formulation comprising the anti-PD-1 antibody at this dose; and about 6 mg to 80 mg, about 80 mg to 200 mg, about 200 mg to 300 mg, or about 300 mg to 600 mg, such as about 6 mg, about 12 mg, about 25 mg, about 35 mg, about 50 mg, about 75 mg, about 125 mg, or about 200 mg of the anti-CTLA4 antibody, or a formulation comprising the anti-CTLA4 antibody at this dose. In some embodiments, the patient is treated with a single dose of the anti-PD-1 antibody and a single dose of the anti-CTLA4 antibody. In some embodiments, the patient is treated with a single dose of a combination of the anti-PD-1 antibody and the anti-CTLA4 antibody.

In some embodiments, 200 mg of the anti-PD-1 antibody and 0.1 mg/kg of the anti-CTLA4 antibody are administered once every 3 weeks. In some embodiments, 200 mg of the anti-PD-1 antibody is administered once every 3 weeks, and 1 mg/kg of the anti-CTLA4 antibody is administered once every 3 weeks. In some embodiments, the patient is relieved of symptoms after a single dose administration. In some embodiments, after a single dose administration, the patient is not relieved of symptoms as expected, and about 50 mg to 600 mg of the anti-PD-1 antibody and about 70 mg to 400 mg of HER2-ADC are separately administered to the patient. In some embodiments, after a single dose administration, the patient is not relieved of symptoms as expected, and a combination of about 50 mg to 600 mg of the anti-PD-1 antibody and about 70 mg to 400 mg of HER2-ADC is administered to the patient. In some embodiments, after a single dose administration, the patient is not relieved of symptoms as expected, and about 50 mg to 600 mg of the anti-PD-1 antibody and about 6 mg to 600 mg of the anti-CTLA4 antibody are separately administered to the patient. In some embodiments, after a single dose administration, the patient is not relieved of symptoms as expected, and a combination of about 50 mg to 600 mg of the anti-PD-1 antibody and about 6 mg to 600 mg of the anti-CTLA4 antibody is administered to the patient.

In some embodiments, the anti-PD-1 antibody (or formulation), the HER2-ADC (or formulation), or the anti-CTLA4 antibody (or formulation) is administered by subcutaneous (s.c.) injection, intraperitoneal (i.p.) injection, parenteral injection, intra-arterial injection, intravenous (i.v.) injection, or the like. In some embodiments, the anti-PD-1 antibody (or formulation) or the HER2-ADC (or formulation) is administered by infusion. In some embodiments, the anti-PD-1 antibody (or formulation) or the HER2-ADC (or formulation) is administered by bolus injection. In some embodiments, the anti-PD-1 antibody (or formulation) or the anti-CTLA4 antibody (or formulation) is administered by infusion. In some embodiments, the anti-PD-1 antibody (or formulation) or the anti-CTLA4 antibody (or formulation) is administered by bolus injection.

In some embodiments, the anti-PD-1 antibody (or formulation), the HER2-ADC (or formulation), or the anti-CTLA4 antibody (or formulation) is administered by intravenous (i.v.) infusion. In some embodiments, the intravenous infusion is performed for about 50 min, about 55 min, about 60 min, about 65 min, about 70 min, about 75 min, about 81 min, about 87 min, about 90 min, about 95 min, or a range between any two of these values (inclusive) or any value therein.

In some embodiments, the anti-PD-1 antibody (or formulation), the therapeutic agent (or formulation), and other treatment methods are used in combination for treating a tumor or cancer, such as chemotherapy, radiotherapy, surgical treatment, and the like.

In another aspect, the present invention discloses use of the anti-PD-1 antibody and the therapeutic agent in the preparation of a drug for treating a tumor or cancer. In some embodiments, the therapeutic agent is selected from an anti-EGRR antibody, an anti-VEGF antibody, an anti-VEGFR2 antibody, an anti-CTLA4 antibody, an anti-PD-L1 antibody, an anti-HER2 antibody, an anti-CD20 antibody, an anti-Trop2 antibody, an anti-TIGIT antibody, an anti-OX40 antibody, an anti-ICOS antibody, an anti-HER2 antibody-drug conjugate, and an anti-Trop2 antibody-drug conjugate. In some embodiments, the drug for treating a tumor or cancer includes an anti-PD-1 antibody and a therapeutic agent. In some embodiments, the anti-PD-1 antibody is an antibody A. In some embodiments, the therapeutic agent is HER2-ADC. In some embodiments, the HER2-ADC is HER2-ADC_{B}. In some embodiments, the therapeutic agent is an anti-CTLA4 antibody. In some embodiments, the anti-CTLA4 antibody is an antibody C.

In another aspect, the present invention further discloses a kit comprising the anti-PD-1 antibody (or formulation), the therapeutic agent (or formulation), and instructions for directing administration of the anti-PD-1 antibody (or formulation) and the therapeutic agent (or formulation) to a patient in need. In some embodiments, the present invention further discloses a kit comprising a composition (or formulation) of an anti-PD-1 antibody and a therapeutic agent, and instructions for directing administration of the composition (or formulation) of the anti-PD-1 antibody and the therapeutic agent to a patient in need.

In another aspect, the present invention further discloses a pharmaceutical composition which comprises an anti-PD-1 antibody and a therapeutic agent and is suitable for injection, such as bolus injection or infusion (drip) pharmaceutical composition. In some embodiments, the pharmaceutical composition comprises at least 0.1% of the anti-PD-1 antibody and 0.1% of the therapeutic agent. The percentage of the antibody and the therapeutic agent may vary and is between about 2% and about 90% by weight of a given dosage form. The amount of the anti-PD-1 antibody and the therapeutic agent in such a therapeutically useful pharmaceutical composition may be an effective amount for administration.

In another aspect, the present invention further discloses a preparation method for the pharmaceutical composition described above, which comprises mixing the anti-PD-1 antibody and the therapeutic agent (or a combination of the anti-PD-1 antibody and the therapeutic agent) described herein with a pharmaceutically acceptable carrier suitable for injection (e.g., water for injection, normal saline, and the like). The procedure of mixing the anti-PD-1 antibody and therapeutic agent described above with a pharmaceutically acceptable carrier are generally known in the art.

In the present invention, the symptoms can be relieved by using the anti-PD-1 antibody (or formulation) and the therapeutic agent (or formulation) in tumor or cancer treatment.

### BRIEF DESCRIPTION OF THE DRAWINGS

FIG. 1 shows the inhibition of the proliferation of tumor cells by the administration of an anti-PD-1 antibody in combination with HER2-ADC.
FIG. 2 shows the change in tumor volume after the administration of an antibody A in combination with an antibody C, wherein the mean body weight is expressed as mean ± SEM.
FIG. 3 shows the weight of tumor on day 27 after the administration of an antibody A and an antibody C, wherein data are expressed as mean ± SEM. Versus G1, the test method of groups except the G5 group is independent sample T test, and the test method of the G5 group is two independent sample tests, Mann-Whitney test, due to the abnormal distribution of data. * denotes P < 0.05, ** denotes P < 0.01, and *** denotes P < 0.001.
FIG. 4 shows the change in body weight of mice after the administration of an antibody A in combination with an antibody C.

### Terms

Unless otherwise specified, each of the following terms shall have the meaning described below.

### Definitions

It should be noted that the term "an" entity refers to one or more of the entities. For example, "an antibody" should be interpreted as one or more antibodies. Thus, the terms "a" (or "an"), "one or more", and "at least one" are be used interchangeably herein.

As used herein, the term "contain" or "comprise" means that the compositions, methods and the like comprise the recited elements, such as components or steps and do not exclude the others. "Consisting essentially of..." means that the compositions and methods exclude other elements that have a fundamental impact on the characteristics of the combination, but do not exclude elements that do not substantially affect the characteristics of the compositions or methods. "Consisting of..." means that elements not specifically listed are excluded.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a particular length of the product. Thus, included within the definition of "polypeptide" are peptides, dipeptides, tripeptides, oligopeptides, "proteins", "amino acid chains", or any other term used to refer to chains of two or more amino acids, and the term "polypeptide" may be used in place of, or alternatively with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage or non-naturally occurring amino acid modification. The polypeptide may be derived from a natural biological source or produced by recombinant techniques, but it is not necessarily translated from a specified nucleic acid sequence, and it may be produced in any manner, including chemical synthesis.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The fact that an amino acid is encoded by different codons is known as "codon degeneracy". Amino acids include natural amino acids and non-natural amino acids. Natural amino acids include alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Conservative amino acid substitution" refers to the substitution of one amino acid residue with another amino acid residue with a side chain (R group) of similar chemical properties (e.g., charge or hydrophobicity). Generally, conservative amino acid substitutions do not substantially alter the functional properties of the protein. Examples of amino acid classes with a side chain of similar chemical properties include: 1) amino acids with an aliphatic side chain: glycine, alanine, valine, leucine, and isoleucine; 2) amino acids with an aliphatic hydroxyl side chain: serine and threonine; 3) amino acids with an amide-containing side chain: asparagine and glutamine; 4) amino acids with an aromatic side chain: phenylalanine, tyrosine, and tryptophan; 5) amino acids with a basic side chain: lysine, arginine, and histidine; and 6) amino acids with an acidic side chain: aspartic acid and glutamic acid.

The number of amino acids in "conservative amino acid substitutions of VL or VH" may be about 1, about 2, about 3, about 4, about 5, about 6, about 8, about 9, about 10, about 11, about 13, about 14, about 15, or a range between any two of these values (inclusive) or any value therein. The number of amino acids in "conservative amino acid substitutions of a heavy light or a light chain" may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

The term "encoding" as applied to a polynucleotide means that a polynucleotide known to "encode" a polypeptide, when in its native state or manipulated by methods well known to those skilled in the art, can produce the polypeptide and a fragment thereof by transcript and/or translation.

The term "recombinant", with regard to a polypeptide or polynucleotide, refers to a polypeptide or polynucleotide that does not occur in nature, and non-limiting examples can be combined to produce a polynucleotide or polypeptide that does not normally occur.

"Homology" or "identity" or "similarity" refers to sequence similarity between two peptides or between two nucleic acid molecules. Homology can be determined by comparing the positions that can be aligned in the sequences. When a position of the compared sequences is occupied by the same base or amino acid, the molecules are homologous at that position. The degree of homology between the sequences is a function of the number of matching or homologous positions shared by the sequences.

"At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

A polynucleotide and a polynucleotide sequence (or polypeptide or antibody sequence) having a certain percentage (e.g., 90%, 95%, 98% or 99%) of "identity" or "sequence identity" to another sequence means that when the sequences are aligned, the percentage of bases (or amino acids) in the sequence are the same. This alignment identity percentage or sequence identity can be determined by visual inspection or using software programs known in the art, such as the software programs described in Ausubel et al. eds., (2007), Current Protocols in Molecular Biology*.* Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above specified percentage identity and encoding polypeptides having identical or similar biological activity.

"Antibody" and "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising at least a portion of an immunoglobulin molecule that has biological activity for binding to an antigen. The antibody and the antigen-binding fragment include, but are not limited to, complementarity determining regions (CDRs), heavy chain variable regions (VHs), light chain variable regions (VLs), heavy chain constant regions (CHs), light chain constant regions (CLs), framework regions (FRs) of a heavy or light chain or a ligand binding portion thereof, or any portion thereof, or at least a portion of a binding protein. The CDR regions include the CDR regions of the light chain (LCDR1-3) and the CDR regions of the heavy chain (HCDR1-3).

The term "antibody" includes a wide variety of polypeptides that can be biochemically distinguished. It will be understood by those skilled in the art that the classes of heavy chains include gamma, mu, alpha, delta, or epsilon (γ, µ, α, δ, or ε), with some subclasses (e.g., γ1-γ4). The nature of this chain determines the "type" of the antibody as IgG, IgM, IgA, IgD or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In some embodiments, the immunoglobulin molecule is an IgG species. These four chains are connected in a "Y" configuration through disulfide bonds, wherein the light chain starts at the opening of "Y" configuration and extends through the variable region to surround the heavy chain.

Antibodies, antigen-binding fragments or derivatives disclosed herein include, but are not limited to, polyclonal, monoclonal, multispecific, fully human, humanized, primatized and chimeric antibodies, single-chain antibodies, epitope-binding fragments (e.g., Fab, Fab' and F(ab')₂), and single-chain Fvs (scFvs).

Light chains can be classified into kappa (κ) or lambda (λ). Each heavy chain may be connected with a x or λ light chain. In general, when an immunoglobulin is produced by a hybridoma, a B cell or a genetically engineered host cell, the light and heavy chains are bound by covalent bonds, and the "tail" portions of the two heavy chains are bound by covalent disulfide bonds or non-covalent bonds. In the heavy chains, the amino acid sequence extends from the N terminus at the forked end of the Y configuration to the C terminus at the bottom of each chain. The immunoglobulin x light chain variable region is Vκ; the immunoglobulin λ light chain variable region is V_{λ}.

The light chain variable region (VL) and the heavy chain variable region (VH) determine the antigen recognition and specificity. The light chain constant region (CL) and the heavy chain constant region (CH) impart important biological properties such as secretion, transplacental movement, Fc receptor binding, and complement fixation. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl terminal of the heavy chain and light chain, respectively.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found in the variable regions of heavy and light chain polypeptides. This particular region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entirety.

CDRs defined according to Kabat and Chothia schemes include overlaps or subsets of amino acid residues when compared to each other. Nevertheless, it is within the scope of the present invention to apply any definition to refer to the CDRs of an antibody or a variant thereof. The exact number of residues comprising a particular CDR will vary according to the sequence and size of the CDR. Those skilled in the art can generally determine which particular residues a CDR comprises based on the variable region amino acid sequence of an antibody.

Kabat et al. also defines a numbering system applicable to the variable region sequence of any antibody. Those of ordinary skill in the art can apply the "Kabat numbering" scheme to any variable region sequence without depending on other experimental data beyond the sequence itself. "Kabat numbering" refers to the numbering scheme proposed by Kabat et al., U.S. Dept. of Health and Human Services in "Sequence of Proteins of Immunological Interest" (1983). EU or Chothia numbering scheme can be also applicable to the antibody.

The term "antibody-drug conjugate" or "ADC" in the present invention refers to an antibody or an antigen-binding fragment thereof chemically linked to one or more chemical drugs (which may optionally be therapeutic or cytotoxic agents). In some embodiments, the ADC comprises an antibody, a cytotoxic or therapeutic agent, and a linker that enables the drug to be linked or conjugated to the antibody. The ADC generally has 1 drug, 2 drugs, 3 drugs, 4 drugs, 5 drugs, 6 drugs, 7 drugs, 8 drugs, 9 drugs, or 10 drugs conjugated to the antibody. Drugs that may be included in the ADC include, but are not limited to: mitotic inhibitors, anti-tumor antibiotics, immunomodulators, vectors for gene therapy, alkylating agents, anti-angiogenic agents, antimetabolites, boron-containing agents, chemoprotectants, hormones, anti-hormonal agents, corticosteroids, photoactive therapeutic agents, oligonucleotides, radionuclide agents, topoisomerase inhibitors, tyrosine kinase inhibitors, and radiosensitizers. In some embodiments, the drug included in the ADC may be a maytansinoid drug. In some embodiments, the drug included in the ADC may be a compound of formula I or a pharmaceutically acceptable salt thereof as described herein. In some embodiments, in the ADC, the antibody is conjugated to the drug through disulfide bonds formed from its own cysteine or a sulfhydrylated amino acid, such as sulfhydrylated lysine.

The terms "alkyl" and "alkylene" as used herein are intended to include both branched and linear saturated aliphatic hydrocarbon groups having the specified number of carbon atoms. For example, the definition of "C1-C6" in "C1-C6 alkyl" includes groups having 1 carbon atom, 2 carbon atoms, 3 carbon atoms, 4 carbon atoms, 5 carbon atoms, or 6 carbon atoms arranged in a linear or branched chain. For example, "C1-C6" specifically includes methyl, ethyl, n-propyl, isopropyl, n-butyl, tert-butyl, isobutyl, pentyl (including 8 isomers), and hexyl (including 23 isomers). The term "cycloalkyl" refers to a monocyclic saturated aliphatic hydrocarbon group having the specified number of carbon atoms. For example, "cycloalkyl" includes cyclopropyl, methyl-cyclopropyl, 2,2-dimethyl-cyclobutyl, 2-ethyl-cyclopentyl, cyclohexyl, and the like.

The term "halogen" as used herein includes fluorine, chlorine, bromine, and iodine.

The term "amino acid side chain" as used herein refers to a substituent that replaces a group (e.g., a hydrogen atom) in an amino acid. For example, a glycine side chain is a substituent formed by substituting one hydrogen atom on a glycine methylene group. Examples of amino acid side chains include, but are not limited to, natural amino acid side chains.

Antibody-drug conjugates can form a wide variety of pharmaceutically acceptable salts, including but not limited to: acid addition salts formed with organic acids including, but not limited to, aliphatic monocarboxylic and dicarboxylic acids, phenyl substituted alkanoic acids, hydroxy alkanoic acids, alkanedioic acids, aromatic acids, aliphatic and aromatic sulfonic acids, amino acids, and the like, e.g., acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like; acid addition salts formed by reaction with inorganic acids including hydrochloric acid, hydrobromic acid, sulfuric acid, nitric acid, phosphoric acid, hydroiodic acid, hydrofluoric acid, phosphorous acid, and the like; and salts with metal ions (e.g., alkali metal ions (e.g., sodium or potassium), alkaline earth metal ions (e.g., calcium or magnesium), or aluminum ions) or with organic bases such as diethanolamine, triethanolamine, N-methylglucamine, and the like. The antibody-drug conjugates described herein include pharmaceutically acceptable salts thereof.

"Treatment" refers to both therapeutic treatment and prophylactic or preventative measures, the purpose of which is to prevent, slow down, ameliorate, or halt undesirable physiological changes or disorders, such as the progression of a disease, including but not limited to, the alleviation of symptoms, the reduction in severity of the disease, the stabilization (i.e., not worsening) of the disease state, the delay or slowing of disease progression, the amelioration or palliation or the alleviation or elimination (whether partial or total) of the disease state, the prolongation of life expectancy as compared to that without the treatment, and the like, whether detectable or undetectable. Patients in need of treatment include patients who already have a condition or disorder, patients who are susceptible to the condition or disorder, or patients who require the prevention of the condition or disorder, or patients who may or are expected to benefit from administration of the antibody or the pharmaceutical composition disclosed herein for detection and diagnostic procedures, and/or treatment.

"Patient" refers to any mammal in need of diagnosis, prognosis or treatment, including humans, dogs, cats, guinea pigs, rabbits, rats, mice, horses, cattle and the like.

"About" refers to a general error range for corresponding values as readily understood by those skilled in the relevant art. In some embodiments, "about" mentioned herein refers to the value described and its range of ± 10%, ± 5% or ± 1%.

"Effective amount" refers to an amount of an active compound or agent that can elicit a biological or medical response in a tissue, system, animal, subject, or human. An effective amount is sought by a researcher, veterinarian, medical doctor or other clinician.

As used herein, the term "in need" means that a patient has been identified as in need of a particular method or treatment. In some embodiments, identification may be made by any diagnostic mode. The patient may be in need of any methods and treatments described herein.

The DNA encoding the antibody can be designed and synthesized based on the amino acid sequence of the antibody described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody coding sequence, a transcription termination signal, and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, or Plncx, pcDNA3.1 (+/-), pcDNA/Zeo (+/-), pcDNA3.1/Hygro(+/-), PSVL, PMSG, pRSVcat, pSV2dhfr, pBC12MI, pCS2, and the like. Commonly used mammalian cells include 293 cells, Cos1 cells, Cos7 cells, CV1 cells, murine L cells, CHO cells, and the like.

"EC₅₀", i.e., concentration for 50% of maximal effect, refers to a concentration that causes 50% of maximal effect.

### DETAILED DESCRIPTION

The technical solution of the present invention will be further illustrated below through specific examples, which are not intended to limit the protection scope of the present invention. Some nonessential modifications and adjustments made by other people according to the concept of the present invention still fall within the protection scope of the present invention.

The materials, reagents, and the like used in the following examples can be commercially available unless otherwise stated.

### Example 1. Preparation Method for Antibodies

1) DNA sequences of the light and heavy chains were constructed based on amino acid sequences of the heavy and light chains of the antibody. The 5' and 3' terminal of the DNA sequences were modified with PCR primers designed to add an appropriate leader sequence to each strand, and then cloned into existing recombinant antibody expression vectors. The correct construction of the vectors was verified by sequencing analysis. The expression vector was introduced into CHO cells for expression, and purified to obtain the antibody. For the preparation method for the antibody A, reference can be made to the patent application with the application number of PCT/CN2020/083954. The amino acid sequences of the antibody A, the antibody B and the antibody C are shown in Table 1, and the nucleic acid sequences of the antibody A and the antibody C are shown in Table 2.

**Table 1. Amino acid sequence**

| Name | SEQ ID NO | Amino acid sequence |
|---|---|---|
| HCDR1 of antibody A | 1 | NYYMYW |
| HCDR2 of antibody A | 2 | GINPSNGGTNFNEKF |
| HCDR3 of antibody A | 3 | ARDYRLDMGFEF |
| LCDR1 of antibody A | 4 | ASKGVSTSGYSYLH |
| LCDR2 of antibody A | 5 | LASYLE |
| LCDR3 of antibody A | 6 | YCQHAYDLPLT |
| VH of antibody A | 7 | QVQLVQSGVEVKKPGASVKVSCKASGYTFTNYYMYWVRQAPGQGLEWM GGINPSNGGTNFNEKFKNRVTLTTDSSTTTAYMELKSLQFDDTAVYYCAARD YRLDMGFEFWGQGTTVTVSS |
| VL of antibody A | 8 | EIVLTQSPATLSLSPGERATLSCRASKGVSTSGYSYLHWYQQKPGQAPRLLIY LASYLESGVPARFSGSGSGTDFTLTISSLEPEDFAVYYCQHAYDLPLTFGGGT KVEIK |
| Heavy chain of antibody A | 9 | QVQLVQSGVEVKKPGASVKVSCKASGYTFTNYYMYWVRQAPGQGLEWM GGINPSNGGTNFNEKFKNRVTLTTDSSTTTAYMELKSLQFDDTAVYYCAARD YRLDMGFEFWGQGTTVTVSSASTKGPSVFPLAPCSRSTSESTAALGCLVKD YFPEPVTVSWNSGALTSGVHTFPAVLQSSGLYSLSSVVTVPSSSLGTKTYTCN VDHKPSNTKVDKRVESKYGPPCPPCPAPEFLGGPSVFLFPPKPKDTLMISRTP EVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEM TKNQVSLTCLVKGFYPSDIAVEWESNGQPENNYKTTPPVLDSDGSFFLYSRL TVDKSRWQEGNVFSCSVMHEALHNHYTQKSLSLSLGK |
| Light chain of antibody A | 10 | EIVLTQSPATLSLSPGERATLSCRASKGVSTSGYSYLHWYQQKPGQAPRLLIY LASYLESGVPARFSGSGSGTDFTLTISSLEPEDFAVYYCQHAYDLPLTFGGGT KVEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVT KSFNRGEC |
| Heavy chain of antibody B | 11 | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRT PEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREE |
| | | |
| Light chain of antibody B | 12 | DIQMTQSPSSLSASVGDRVTITCRASQDVNTAVAWYQQKPGKAPKLLIYSAS FLYSGVPSRFSGSRSGTDFTLTISSLQPEDFATYYCQQHYTTPPTFGQGTKVEI KRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSF NRGEC |
| Heavy chain of antibody C | 15 | QVQLVESGGGVVQPGRSLRLSCAASGFTFSSYTMHWVRQAPGKGLEWVTF ISYDGNNKYYADSVKGRFTISRDNSKNTLYLQMNSLRAEDTAIYYCARTGW LGPFDYWGQGTLVTVSSASTKGPSVFPLAPSSKSTSGGTAALGCLVKDYFPE PVTVSWNSGALTSGVHTFPAVLQSSGLYSLSSVVTVPSSSLGTQTYICNVNH KPSNTKVDKRVEPKSCDKTHTCPPCPAPELLGGPSVFLFPPKPKDTLMISRTP EVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSRDELT KNQVSLTCLVKGFYPSDIAVEWESNGQPENNYKTTPPVLDSDGSFFLYSKLT VDKSRWQQGNVFSCSVMHEALHNHYTQKSLSLSPGK |
| Light chain of antibody C | 16 | EIVLTQSPGTLSLSPGERATLSCRASQSVGSSYLAWYQQKPGQAPRLLIYGAF SRATGIPDRFSGSGSGTDFTLTISRLEPEDFAVYYCQQYGSSPWTFGQGTKVEI KRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSF NRGEC |

**Table 2. Nucleic acid sequences of antibody A and antibody C**

| Name | SEQ ID NO | Nucleic acid sequence |
|---|---|---|
| Heavy chain of antibody A | 13 | caggtgcagctggtgcagtccggcgtggaggtgaagaagcctggcgccagcgtgaaggtgtcctgtaaggccagcggctac accttcaccaattactatatgtattgggtgcggcaggcccccggccagggactggagtggatgggaggcatcaatcccagcaa cggcggcaccaacttcaatgagaagtttaagaaccgggtgaccctgaccaccgatagcagcaccaccaccgcttacatggag ctgaagagcctgcagtttgacgataccgctgtgtactattgcgctgcccgggattacaggctggacatgggcttcgagttctggg gccagggcaccaccgtgaccgtgtccagcgctagcaccaagggcccttccgtgttccccctggccccctgtagccggtccac ctctgagagcaccgctgctctgggctgtctggtgaaggattactttcccgaaccggtgaccgtgtcatggaactccggggctctg acatccggtgtccacacttttcctgcagtgctgcagtcatccggcctgtacagcctgagctctgtggtcacagtcccaagttcatc cctgggaaccaagacatatacttgcaacgtggatcataaacccagcaatactaaggtcgacaaacgagtggagtctaagtacg gaccaccttgcccaccatgtccagcacctgagttcctgggaggaccaagcgtgttcctgtttcctccaaagcctaaagataccct gatgatcagtcggactcccgaggtcacctgcgtggtcgtggacgtgtcccaggaggaccctgaagtccagttcaactggtacg tggacggcgtcgaagtgcacaatgctaagacaaaacctcgagaggaacagtttaactccacataccgtgtcgtgagcgtcctg actgtgctgcatcaggattggctgaacggcaaggagtataagtgcaaagtgagcaataagggactgccaagctctatcgagaa aactatttctaaggctaaaggacagcctagggaaccacaggtgtacaccctgccccctagtcaggaggaaatgactaagaacc aggtctcactgacctgtctggtgaaagggttctatccttcagatattgcagtggagtgggaatccaatggtcagccagagaacaa ttacaagacaactccacccgtgctggacagcgatgggtctttctttctgtattctagactgaccgtggacaaaagtcgctggcagg agggtaatgtcttttcttgtagtgtgatgcacgaagccctgcacaaccactacactcagaaaagcctgtcactgtccctgggtaaa |
| Light chain of antibody A | 14 | gagatcgtgctgacccagtcccccgctaccctgagcctgtcccccggagagcgggctaccctgtcttgtcgggcctccaaggg cgtgagcaccagcggatactcctatctgcactggtaccagcagaagcccggccaggctcccaggctgctgatctacctggctt cctacctggagagcggcgtgcccgctaggtttagcggcagcggcagcggaaccgatttcaccctgaccatcagctccctgga gcccgaggattttgccgtgtactactgccagcacgcttacgacctgcccctgacctttggcggcggcaccaaggtggagatca agcgtacggtggctgcaccatctgtcttcatcttcccgccatctgatgagcagttgaaatctggaactgcctctgttgtgtgcctgc tgaataacttctatcccagagaggccaaagtacagtggaaggtggataacgccctccaatcgggtaactcccaggagagtgtc acagagcaggacagcaaggacagcacctacagcctcagcagcaccctgacgctgagcaaagcagactacgagaaacacaa agtctacgcctgcgaagtcacccatcagggcctgagctcgcccgtcacaaagagcttcaacaggggagagtgt |
| Heavy chain of antibody C | 17 | caggtccagctggtcgaatctggcggcggcgtcgtccagcctggaagatctctgagactgtcttgcgctgcttctggctttacctt ctcttcttacacaatgcattgggtcagacaggctcctggtaaaggtctggagtgggtcacattcatctcttacgacggcaacaaca agtactacgccgattctgtcaagggcagattcaccatctccagagacaactccaagaacaccctgtacctgcagatgaactctct gagagctgaggataccgctatctactattgcgctagaacaggctggctgggaccttttgattactggggacagggaacactggt cacagtctcttctgcttctaccaaaggaccttctgtctttcctctggctccttcttctaagtctacctctggcggaacagctgctctgg gttgtctggtcaaggattacttccctgaaccggtgacagtgtcttggaattctggagctctgacctcaggagtccatacatttcctg ctgtcctgcagtcttctggcctgtattctctgtcctctgtggtgacagtcccttcttcttctctgggaacacagacctacatctgcaac gtcaaccacaagccttccaacaccaaggtcgacaagagagtggagcctaagtcttgcgacaagacacatacatgtccaccgtg cccagcacctgaactcctggggggaccgtcagtcttcctcttccccccaaaacccaaggacaccctcatgatctcccggaccc |
| | | ctgaggtcacatgcgtggtggtggacgtgagccacgaagaccctgaggtcaagttcaactggtacgtggacggcgtggaggt gcataatgccaagacaaagccgcgggaggagcagtacaacagcacgtaccgtgtggtcagcgtcctcaccgtcctgcacca ggactggctgaatggcaaggagtacaagtgcaaggtctccaacaaagccctcccagcccccatcgagaaaaccatctccaaa gccaaagggcagccccgagaaccacaggtgtacaccctgcccccatcccgggatgagctgaccaagaaccaggtcagcct gacctgcctggtcaaaggcttctatcccagcgacatcgccgtggagtgggagagcaatgggcagccggagaacaactacaa gaccacgcctcccgtgctggactccgacggctccttcttcctctacagcaagctcaccgtggacaagagcaggtggcagcag gggaacgtcttctcatgctccgtgatgcatgaggctctgcacaaccactacacgcagaagagcctctccctgtctccgggtaaa |
| Light chain of antibody C | 18 | gaaattgtcctgacacagtctcctggaacactgtctctgtctcctggagagagagctacactgtcttgtagggcttctcagtctgtg ggatcttcttacctggcttggtatcagcagaaacctggacaggctcctagactgctgatctacggcgctttttctagagctaccgg aatccctgacagattctctggatctggctctggaaccgatttcaccctgaccatctctagactggaacctgaggactttgctgtcta ctactgtcagcagtacggctcttctccttggacatttggacagggaaccaaggtcgagatcaagcgtacggtggctgcaccatct gtcttcatcttcccgccatctgatgagcagttgaaatctggaactgcctctgttgtgtgcctgctgaataacttctatcccagagagg ccaaagtacagtggaaggtggataacgccctccaatcgggtaactcccaggagagtgtcacagagcaggacagcaaggaca gcacctacagcctcagcagcaccctgacgctgagcaaagcagactacgagaaacacaaagtctacgcctgcgaagtcaccc atcagggcctgagctcgcccgtcacaaagagcttcaacaggggagagtgt |

2) The antibody C was prepared from CHO-BAT-KF cells, purified and determined for glycoform content. The results are shown in Table 3.

**Table 3. Percentage content of part of glycoforms**

| Glycoform classification | | Antibody C |
|---|---|---|
| Sialylated glycoform | G1S1 | 0.04 |
| | G1FS1 | / |
| | G2S1 | 0.17 |
| | G2S1' | 0.1 |
| | G2FS1 | / |
| | G2FS1' | / |
| | G2S2 | 0.14 |
| | G2FS2 | / |
| High mannose glycoform | Man5 | 1.08 |
| | Man6 | / |
| | Man8 | 0.1 |

### Example 2. Preparation of HER2-ADC_{B}

For the preparation of HER2-ADC_{B}, reference can be made to Examples 1-3 and Example 9 of the patent application with the publication number of WO2014094527A.

### Example 3. Inhibition of Proliferation of NCI-N87 Cells (Human Gastric Cancer Cells) by In Vitro Administration of Antibody A in Combination with HER2-ADC_{B}

The concentration of the antibody A was fixed (25 µg/mL), and the concentration of HER2-ADC_{B} was changed (starting from 10 nM, 3-fold gradient dilution). PBMC cells (Leide Biosciences Co., Ltd.) were added, and SEB (staphylococcal enterotoxin B, Academy of Military Medical Sciences SL008) was simultaneously added to activate the PBMC cells. After 72 h of culturing, the inhibitory effect of the *in vitro* combination therapy on the proliferation of HER2 and PD-L1 double-positive cells by a CCK method (CCK8 kit from Dojindo). The specific procedures were as follows:
1) 96-well plate cell plating: NCI-N87 cells were adjusted to a density of 2.0×10⁵ cells/mL, and plated at 10000 cells/well, with each well of 50 µL.
2) After 3-5 h of cell culturing, PBMC cells were added, and adjusted to a density of 8.0×10⁵ cells/mL (ratio of PBMC cells to NCI-N87 cells of 4:1) with a medium containing 100 ng/mL SEB, with the volume of 50 µL.
3) HER2-ADC_{B} was serially diluted in a 3-fold gradient from 10 nM, then a total of 9 gradients (10 nM, 3.3 nM, 1.1 nM, 0.37 nM, 0.12 nM, 0.04 nM, 0.01 nM, 0.004 nM, and 0.001 nM) were obtained, and the well 10 was set at a concentration of 0, with each well of 50 µL. 25 µg/mL antibody A was added to the top three rows at 50 µL/well, and medium was added to the bottom three rows at 50 µL/well as a control, with the arrangement shown as the following table. The antibody A and HER2-ADC_{B} were diluted with RPMI1640 (Gibco) + 10% FBS (Excell Bio).
4) The cells were cultured in an incubator at 37 °C with 5% CO₂ for about 72 h, the supernatant was removed, and a medium containing 10% CCK8 was added.
5) The cells were cultured at 37 °C for 4 h, and the plate was read for absorbance on a microplate reader at a wavelength of 450 nm.

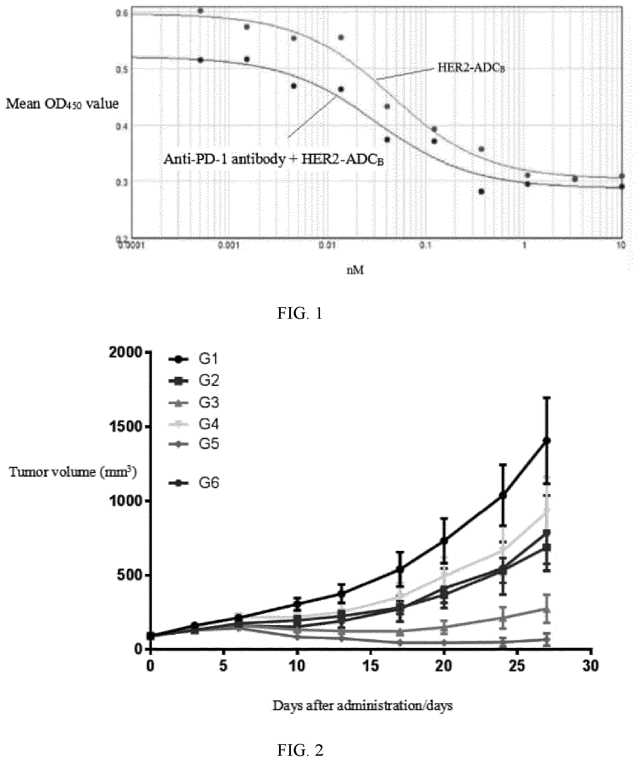

The results are shown in FIG. 1, which shows that in the presence of PBMC, the antibody A (anti-PD-1 antibody) can enhance the proliferation inhibitory effect of HER2-ADC_{B} on the proliferation of NCI-N87 tumor cells to a certain extent. By calculation, EC₅₀ was 0.032 nM for the administration of antibody A in combination with HER2-ADC_{B}, and EC₅₀ was 0.045 nM for the administration of HER2-ADC_{B} alone.

### Example 4. Inhibition of Proliferation of Cancer Cells by Administration of Antibody A in Combination with Antibody C

In this example, the efficacy of the antibody A and the antibody C (expressed by CHO-BAT-KF cells) in an immune checkpoint colon cancer tumor model of humanized mouse C57BL/6-hPD1/hCTLA4 subcutaneously inoculated with MC38 was evaluated.

### 1) Tumor cell inoculation

Mouse colon cancer MC38 cells (Jiangsu GemPharmatech Co., Ltd.) were thawed, and MC38 cells (Jiangsu GemPharmatech Co., Ltd.) in logarithmic phase were collected. The culture medium was removed, and the cells were washed twice with PBS, and inoculated (the cell survival rates before and after tumor bearing were 99.6% and 97.8%, respectively) in an amount of 1×10⁶/100 µL/mouse by intraperitoneal injection at the axilla.

### 2) Grouping and administration

On day 6 after the inoculation, when the mean tumor volume reached 89.19 mm³, 60 female mice were randomly divided into 6 groups of 10 mice per group based on the tumor volume. The day of grouping was defined as D0, and the administration was started on D0. The administration regimen for the groups is shown in Table 4, with the administration performed on D0, D4, D7, D11, D14, and D18.

### 3) Experimental observation and data acquisition

After cell inoculation, the effect of tumors on the normal behavior of the animals was routinely monitored weekly. Specific indicators include mobility, food and water intake, weight gain or loss, eyes, hair, and other abnormal conditions of mice.

After the start of administration, the mice were observed for tumor size and determined for body weight on D0, D3, D6, D10, D13, D17, D20, D24, and D27. The tumor volume was calculated as follows: tumor volume (mm³) = 0.5 × (long diameter of tumor × short diameter of tumor).

### 4) Statistics

The results of the tumor volume, body weight, tumor weight, and the like of each group of mice were expressed as mean ± standard error (mean ± SEM). Different administration groups were compared with the control group for any significant difference by the independent sample T test. Data were analyzed using SPSS. P < 0.05 was defined as a significant difference.

TGItv (tumor growth inhibition of relative tumor volume) calculation formula:
RTVₙ = Vₙₜ/Vₙ₀, wherein Vₙₜ: tumor volume of mice numbered n on day t, Vₙ₀: tumor volume of mice numbered n on day 0, and RTVₙ: relative tumor volume of mice numbered n on day t;
TGItv = (1 - (mean RTV_{administartion group}) / (mean RTV_{control group})) × 100%, wherein mean RTV_{administration group}: mean RTV of the administration group, and mean RTV_{control group}: mean RTV of control group.

TGItw (tumor growth inhibition of tumor weight) calculation formula:
TGItw = (1 - (mean TW_{administration group})/(mean TW_{control group})) × 100%, wherein mean TW_{administration group}: mean value of tumor weight of mice in the administration group at the time of endpoint treatment, and mean TW_{control group}: mean valeu of tumor weight of mice in the control group at the time of endpoint treatment.

**Table 4. Administration regimen**

| Group | N | Administration group | Dose (mg/kg) | Route | Frequency | Number of actual administrations |
|---|---|---|---|---|---|---|
| G1 | 10 | Normal saline | - | i.p. | BIWx3 | 6 |
| G2 | 10 | Antibody A | 0.3 | i.p. | BIWx3 | 6 |
| G3 | 10 | Antibody C | 0.1 | i.p. | BIWx3 | 6 |
| G4 | 10 | Antibody C | 0.03 | i.p. | BIWx3 | 6 |
| G5 | 10 | Antibody A | 0.3 | i.p. | BIWx3 | 6 |
| | | Antibody C | 0.1 | i.p. | BIWx3 | 6 |
| G6 | 10 | Antibody A | 0.3 | i.p. | BIWx3 | 6 |
| | | Antibody C | 0.03 | i.p. | BIWx3 | 6 |

| | | | | | | |
|---|---|---|---|---|---|---|
| Note: N represents the number of animals; BIW × 3W represents twice weekly for 3 weeks; intraperitoneal administration is performed for 6 times; the groups G5 and G6 are combination therapy group, the antibody A is administered first, and the antibody C is administered 2 h later, at a concentration not doubled and consistent with that of the monotherapy group. | | | | | | |

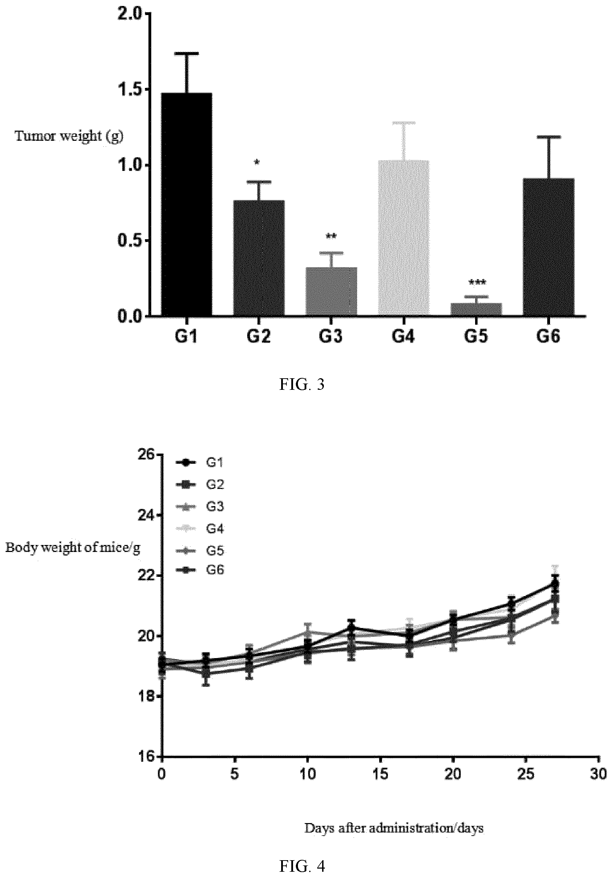

As shown in FIGs. 2 and 3 and Tables 5 and 6, the monotherapy antibody A had a significant anti-tumor effect, the high-dose group of antibody C showed a significant anti-tumor effect, and the antibody C exhibited a certain dose-dependent effect; the high-dose combination therapy group of the antibody A and the antibody C had a significant anti-tumor effect, which was superior to that of the monotherapy antibody A and the monotherapy antibody C (no complete tumor regression was found in mice in G1 and G2 groups, 2 mice in each of G3 and G6 groups had complete tumor regression, 1 mouse in G4 had complete tumor regression, and 6 mice in G5 had complete tumor regression). As shown in FIG. 4, no significant difference was found in the body weight of the mice between the groups, and no mice was weighed less than 10% of the body weight of the mice on the day of the grouping during the experiment, indicating that the mice were well tolerated by the drug in the current system.

**Table 5. Tumor growth inhibition of tumor volume (TGItv) of different groups**

| Group | D0 | D3 | D6 | D10 | D13 | D17 | D20 | D24 | D27 |
|---|---|---|---|---|---|---|---|---|---|
| G1 | - | - | - | - | - | - | - | - | - |
| G2 | 0.00% | 19.83% | 19.09% | 36.21% | 39.78% | 46.98% | 49.13% | 48.07% | 50.31% |
| G3 | 0.00% | 16.98% | 25.29% | 56.60% | 66.59% | 76.24% | 78.42% | 78.00% | 78.97% |
| G4 | 0.00% | 5.38% | 0.18% | 29.93% | 35.03% | 37.42% | 35.25% | 38.27% | 36.30% |
| G5 | 0.00% | 20.20% | 34.33% | 73.83% | 81.09% | 92.35% | 94.56% | 95.96% | 95.91% |
| G6 | 0.00% | 18.55% | 26.71% | 50.69% | 48.97% | 48.53% | 42.41% | 46.02% | 42.40% |

**Table 6. Statistical analysis of tumor growth inhibition of tumor weight (TGItw) and P value (vs G1) of different groups on day 27**

| Group | Tumor weight (g) | TGItw | P value |
|---|---|---|---|
| G1 | 1.4662±0.2701 | - | - |
| G2 | 0.7601±0.1302 | 48.16% | 0.030* |
| G3 | 0.3144±0.1050 | 78.56% | 0.002** |
| G4 | 1.0236±0.2542 | 30.19% | 0.248 |
| G5 | 0.0796±0.0500 | 94.57% | < 0.001*** |
| G6 | 0.9008±0.2827 | 38.57% | 0.165 |

## Claims

1. A method for treating a tumor or cancer, comprising administering to a patient in need an effective amount of an anti-PD-1 antibody and a therapeutic agent, wherein
the anti-PD-1 antibody comprises an HCDR1 set forth in SEQ ID NO: 1, an HCDR2 set forth in SEQ ID NO: 2, an HCDR3 set forth in SEQ ID NO: 3, an LCDR1 set forth in SEQ ID NO: 4, an LCDR2 set forth in SEQ ID NO: 5, and an LCDR3 set forth in SEQ ID NO: 6.

2. The method according to claim 1, wherein the anti-PD-1 antibody comprises a heavy chain variable region comprising a sequence set forth in SEQ ID NO: 7, a sequence having at least 80% identity to the sequence set forth in SEQ ID NO: 7, or an amino acid sequence having one or more conservative amino acid substitutions as compared to the sequence set forth in SEQ ID NO: 7; and/or
the anti-PD-1 antibody comprises a light chain variable region comprising a sequence set forth in SEQ ID NO: 8, a sequence having at least 80% identity to the sequence set forth in SEQ ID NO: 8, or an amino acid sequence having one or more conservative amino acid substitutions as compared to the sequence set forth in SEQ ID NO: 8.

3. The method according to claim 1, wherein the anti-PD-1 antibody comprises a heavy chain variable region comprising a sequence set forth in SEQ ID NO: 7, and a light chain variable region comprising a sequence set forth in SEQ ID NO: 8.

4. The method according to claim 1, wherein the anti-PD-1 antibody comprises a heavy chain comprising a sequence set forth in SEQ ID NO: 9, a sequence having at least 80% identity to the sequence set forth in SEQ ID NO: 9, or an amino acid sequence having one or more conservative amino acid substitutions as compared to the sequence set forth in SEQ ID NO: 9; and/or
the anti-PD-1 antibody comprises a light chain comprising a sequence set forth in SEQ ID NO: 10, a sequence having at least 80% identity to the sequence set forth in SEQ ID NO: 10, or an amino acid sequence having one or more conservative amino acid substitutions as compared to the sequence set forth in SEQ ID NO: 10.

5. The method according to claim 4, wherein the anti-PD-1 antibody comprises a heavy chain comprising a sequence set forth in SEQ ID NO: 9, and a light chain comprising a sequence set forth in SEQ ID NO: 10.

6. The method according to any one of claims 1-5, wherein the therapeutic agent is selected from antibodies or antibody-drug conjugates directed against the following targets: EGFR, VEGF, VEGFR2, CTLA4, PD-L1, HER2, CD20, Trop2, Lag3, TIGIT, CD27, OX40, ICOS, BTLA, TIM3, BCMA, c-MET, and TAA-1/2/3.

7. The method according to any one of claims 1-5, wherein the therapeutic agent is an antibody-drug conjugate of formula II or a pharmaceutically acceptable salt: 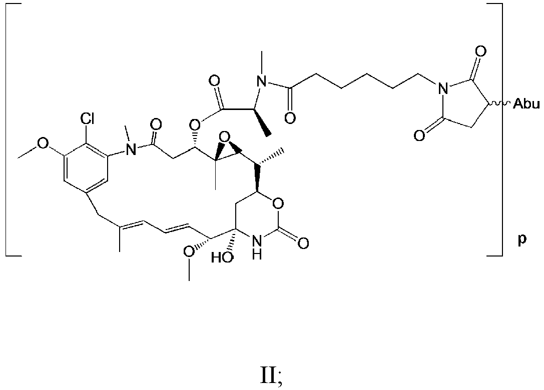
wherein, Abu is an anti-HER2 antibody, and p is selected from 1-10; preferably, p is 3.3-3.7; or
Abu is an anti-Trop2 antibody, and p is selected from 1-10; preferably, p is 2-3.

8. The method according to claim 7, wherein the anti-HER2 antibody comprises a heavy chain comprising a sequence set forth in SEQ ID NO: 11, a sequence having at least 80% identity to the sequence set forth in SEQ ID NO: 11, or an amino acid sequence having one or more conservative amino acid substitutions as compared to the sequence set forth in SEQ ID NO: 11; and/or
the anti-HER2 antibody comprises a light chain comprising a sequence set forth in SEQ ID NO: 12, a sequence having at least 80% identity to the sequence set forth in SEQ ID NO: 12, or an amino acid sequence having one or more conservative amino acid substitutions as compared to the sequence set forth in SEQ ID NO: 12.

9. The method according to claim 8, wherein the anti-HER2 antibody comprises a heavy chain comprising a sequence set forth in SEQ ID NO: 11, and a light chain comprising a sequence set forth in SEQ ID NO: 12.

10. The method according to any one of claims 1-6, wherein the anti-PD-1 antibody is administered at an effective amount of 50 mg to 600 mg per treatment cycle.

11. The method according to claim 7, wherein the anti-HER2 antibody-drug conjugate is administered at an effective amount of 70 mg to 400 mg per treatment cycle.

12. The method according to any one of claims 1-6, wherein the anti-PD-1 antibody is administered at a dose of 1-10 mg/kg each time.

13. The method according to claim 7, wherein the anti-HER2 antibody-drug conjugate is administered at a dose of 1-6 mg/kg each time.

14. The method according to any one of claims 1-6, wherein the therapeutic agent is an anti-TIGIT antibody.

15. The method according to claim 14, wherein the anti-CTLA4 antibody comprises a heavy chain comprising a sequence set forth in SEQ ID NO: 15, a sequence having at least 80% identity to the sequence set forth in SEQ ID NO: 15, or an amino acid sequence having one or more conservative amino acid substitutions as compared to the sequence set forth in SEQ ID NO: 15; and/or
the anti-CTLA4 antibody comprises a light chain comprising a sequence set forth in SEQ ID NO: 16, a sequence having at least 80% identity to the sequence set forth in SEQ ID NO: 16, or an amino acid sequence having one or more conservative amino acid substitutions as compared to the sequence set forth in SEQ ID NO: 16.

16. The method according to claim 15, wherein the anti-CTLA4 antibody comprises a heavy chain comprising a sequence set forth in SEQ ID NO: 15, and a light chain comprising a sequence set forth in SEQ ID NO: 16.

17. The method according to any one of claims 14-16, wherein the anti-CTLA4 antibody is expressed by an α-(1,6)-fucosyltransferase gene knock-out CHO cell line.

18. The method according to claim 6, wherein the anti-CTLA4 antibody is administered at an effective amount of 6 mg to 600 mg per treatment cycle.

19. The method according to claim 6, wherein the anti-CTLA4 antibody is administered at a dose of 0. 1-10 mg/kg each time.

20. The method according to any one of claims 1-19, wherein the patient is subjected to administration for one treatment cycle of 1 week, 2 weeks, 3 weeks, 4 weeks, 1 month, 5 weeks, 6 weeks, or 7 weeks.

21. The method according to any one of claims 1-20, wherein the patient is treated for a plurality of treatment cycles until conditions are alleviated and no treatment is required.

22. The method according to any one of claims 1-20, wherein the patient is subjected to administration by intravenous infusion.

23. A kit comprising an anti-PD-1 antibody, a therapeutic agent, and instructions for directing administration of the PD-1 antibody and the therapeutic agent to a patient in need, wherein
the anti-PD-1 antibody comprises an HCDR1 set forth in SEQ ID NO: 1, an HCDR2 set forth in SEQ ID NO: 2, an HCDR3 set forth in SEQ ID NO: 3, an LCDR1 set forth in SEQ ID NO: 4, an LCDR2 set forth in SEQ ID NO: 5, and an LCDR3 set forth in SEQ ID NO: 6.

24. A pharmaceutical composition comprising an anti-PD-1 antibody and a therapeutic agent, wherein the anti-PD-1 antibody comprises an HCDR1 set forth in SEQ ID NO: 1, an HCDR2 set forth in SEQ ID NO: 2, an HCDR3 set forth in SEQ ID NO: 3, an LCDR1 set forth in SEQ ID NO: 4, an LCDR2 set forth in SEQ ID NO: 5, and an LCDR3 set forth in SEQ ID NO: 6.

25. The kit according to claim 23 or the pharmaceutical composition according to claim 24, wherein the therapeutic agent is selected from antibodies or antibody-drug conjugates directed against the following targets: EGFR, VEGF, VEGFR2, CTLA4, PD-L1, HER2, CD20, Trop2, Lag3, TIGIT, CD27, OX40, ICOS, BTLA, TIM3, BCMA, c-MET, and TAA-1/2/3.
